(19) Europäisches Patentamt
European Patent Office
Office européen des brevets

(11) EP 1 634 877 B1

(12) EUROPEAN PATENT SPECIFICATION

(45) Date of publication and mention of the grant of the patent:
**22.06.2011 Bulletin 2011/25**

(21) Application number: **04254939.4**

(22) Date of filing: **17.08.2004**

(51) Int Cl.:
*C07D 307/34* (2006.01) *C07D 307/60* (2006.01)
*C07D 207/44* (2006.01) *C07D 207/46* (2006.01)
*A61K 31/341* (2006.01) *A61K 31/4015* (2006.01)
*A61K 36/06* (2006.01) *C12N 1/14* (2006.01)

(54) **Mixture and compounds from mycelia of antrodia camphorata and use thereof**

Mischung und Verbindungen von Mycelien von Antrodia Camphorata und deren Verwendung

Mélange et composés de mycélions d’Antrodia Camphorata et leur usage

(84) Designated Contracting States:
**AT BE BG CH CY CZ DE DK EE ES FI FR GB GR HU IE IT LI LU MC NL PL PT RO SE SI SK TR**

(43) Date of publication of application:
**15.03.2006 Bulletin 2006/11**

(73) Proprietor: **Simpson Biotech Co., Ltd.**
**Taoyuan Country 333 (TW)**

(72) Inventors:
- **Hattori, Massao**
  **Toyama 930-0194 (JP)**
- **Sheu, Chia-Chin**
  **Taoyuan Country 333, Taiwan,R.O.C. (TW)**

(74) Representative: **Sexton, Jane Helen**
**J.A. Kemp & Co.**
**14 South Square**
**Gray’s Inn**
**London WC1R 5JJ (GB)**

(56) References cited:
**GB-A- 1 560 464**
**US-A- 4 188 398**
**US-A1- 2003 113 297**
**US-A1- 2003 148 517**
**US-A1- 2004 092 000**

- **NAKAMURA, NORIO ET AL: "Five new maleic and succinic acid derivatives from the mycelium of Antrodia camphorata and their cytotoxic effects on LLC tumor cell line" JOURNAL OF NATURAL PRODUCTS, CODEN: JNPRDF; ISSN: 0163-3864, vol. 67, no. 1, January 2004 (2004-01), pages 46-48, XP002316113**
- **TOMINAGA, YOSHINORI ET AL: "Synthesis of methylthiomaleimides for the preparation of pyridazines and related compounds" JOURNAL OF HETEROCYCLIC CHEMISTRY , 39(3), 571-591 CODEN: JHTCAD; ISSN: 0022-152X, 2002, XP002316114**
- **WIJNBERG, J. B. P. A. ET AL: "A regioselective reduction of gem-disubstituted succinimides" TETRAHEDRON , 34(2), 179-87 CODEN: TETRAB; ISSN: 0040-4020, 1978, XP002316115**
- **MAU ET AL.: "Antioxidant properties of methanolic extracts from two kinds of Antrodia camphorata mycelia" FOOD CHEMISTRY, vol. 86, June 2004 (2004-06), pages 25-31, XP002316116**
- **DAI ET AL.: "The Protection of Antrodia camphorata against Acute Hepatotoxicity" JOURNAL OF FOOD AND DRUG ANALYSIS, vol. 11, no. 3, 2003, pages 177-185, XP002316117**
- **HSEU ET AL.: "Protection of oxidative damage by aqueous extract from Antrodia camphorata mycelia in normal human erythrocytes" LIFE SCIENCES, vol. 71, 2002, pages 469-482, XP002316118**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to novel mixture and compounds from mycelium of *Antrodia camphorata* and the use thereof. The present invention relates to the composition or mycelium comprising the compounds of the invention.

### BACKGROUND OF THE INVENTION

The fruiting body of *Antrodia camphorata* (Polyporaceae, Aphyllophorales) is well known in Taiwan as a traditional Chinese medicine. It grows only on the inner heartwood wall of the endemic evergreen *Cinnamomun kanehirai* (Hay) (Lauraceae) in Taiwan. It is rare and has not been cultivated. The fruiting bodies have been used for treating of food and drug intoxication, diarrhea, abdominal pain, hypertension, itchy skin, and liver cancer. Very few biological activity studies have been reported hitherto.

*Antrodia camphorata*, also known as "niu-chang-chih" or "niu-chang-ku" in Taiwan, was recently reported as a new fungus species characterized by the cylindrical shape of its basidiospores appearing in fruiting bodies, weakly amyloid skeletal hyphae, bitter taste and light cinnamon resupinate to pileate basidiocarps, as well as chlamydospores and anthroconidia in pure culture. The growth of this new fungus species is extremely slow and restricted to an endemic tree species, Cinnamomum kanehirai Hay (Lauraceae), as the only host. The detailed characterization and taxonomic position of *Antrodia camphorata* were described in Wu, S.-H., et al., Antrodia cinnamomea ("niu-chang-chih"), New combination of a medicinal fungus in Taiwan, Bot. Bull. Acad. Sin. 38: 273-275 (1997).

In Taiwanese folk medicine, the fruiting bodies of *Antrodia camphorata* are believed to have certain medical effects. According to the traditional way, the fruiting bodies are ground into dry powder or stewed with other herbal drugs for oral uptake to treat conditions caused by poisoning, diarrhea, abdominal pain, hypertension, skin itches and liver cancer. However, few pharmacological or clinical studies in these aspects have appeared in literature to date. Because of the stringent host specificity and rarity in nature, as well as the failure of artificial cultivation, "niu-chang-chih" is very expensive in Taiwan. In recent years, the fruiting bodies of this fungus with high quality have been sold at an extremely high price of around U.S.$ 15,000 per kg.

Further references can be found in the following documents:

US 2004/092000; US 2003/113297; US 2003/148517; GB-A-1 560 464; US-A-4 188 398;

NAKAMURA, NORIO ET AL, JOURNAL OF NATURAL PRODUCTS, vol. 67, no. 1, January 2004, pages 46-48;

TOMINAGA, YOSHINORI ET AL, JOURNAL OF HETEROCYCLIC CHEMISTRY, 39(3), 571-59;

WIJNBERG, J. B. P. A. ET AL: , TETRAHEDRON , 34(2), 179-87, 1978;

MAU ET AL, FOOD CHEMISTRY, vol. 86, June 2004, pages 25-31;

DAI ET AL., JOURNAL OF FOOD AND DRUG ANALYSIS, vol. 11, no. 3, 2003, pages 177-185;

HSEU ET AL., LIFE SCIENCES, vol. 71, 2002, pages 469-482.

### SUMMARY OF THE INVENTION

Accordingly, an object of the present invention is to provide a compound having the formula

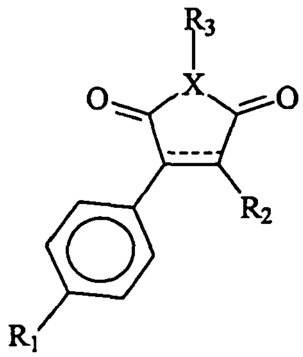

its tautomeric forms, its stereoisomers, its pharmaceutically acceptable salts, or its pharmaceutically acceptable solvates, wherein

X is N or O;

$R_1$ is $C_{1-10}$ alkyloxy, $Z_{2-10}$ alkenyloxy, or $C_{2-10}$ alkynyloxy;

$R_2$ is H, $C_{1-10}$ alkyl, $C_{2-10}$ alkenyl or $C_{2-10}$ alkynyl;

$R_3$ is absent, H, hydroxyl or $C_{1-10}$ alkyl;

----- denotes a single or double bond;

provided that

if X is O, $R_3$ is absent;

if ----- denotes a single bond, the compound has the formula:

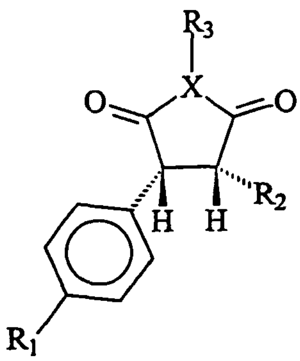

,

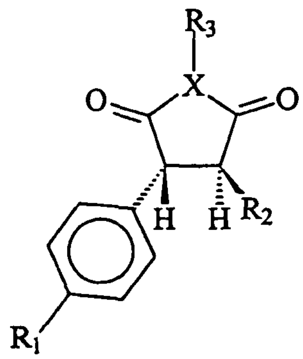

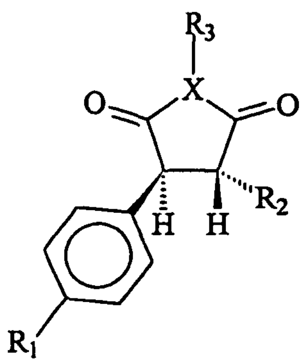

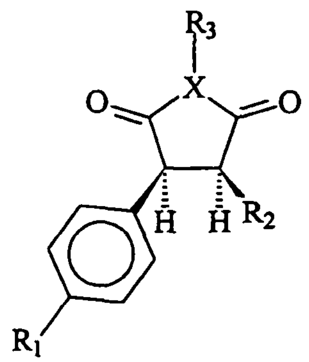

or

,

for use in a method of:

(a) decreasing blood pressure;
(b) increasing high density lipoprotein;
(c) central cholinergic agonism;
(d) providing hepatoprotection against D-galactosamine or Hepatitis C virus; or
(e) treating inflammation.

Another object of the present invention is to provide a mixture from mycelium of *Antrodia camphorate* for:

(a) decreasing blood pressure;
(b) increasing high density lipoprotein;
(c) central cholinergic agonism;
(d) hepatoprotection against D-galactosamine or Hepatitis C virus; or
(e) treating inflammation,

wherein the mycelium has been previously prepared according to submerged liquid fermentation and wherein the mixture is prepared from water or organic solvent extract of said mycelium.

Further object of the present invention is to provide a pharmaceutical composition comprising:

(i) a compound having the formula

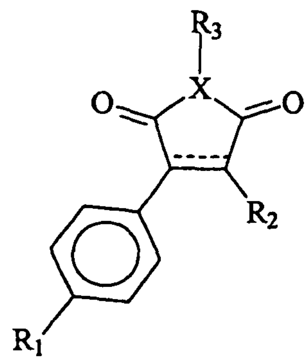

its tautomeric forms, its stereoisomers, its pharmaceutically acceptable salts, or its pharmaceutically acceptable solvates,
wherein
X is N or O;
$R_1$ is $C_{1-10}$ alkyloxy, $C_{2-10}$ alkenyloxy, or $C_{2-10}$ alkynyloxy;
$R_2$ is H, $C_{1-10}$ alkyl, $C_{2-10}$ alkenyl or $C_{2-10}$ alkynyl;
$R_3$ is absent, H, hydroxyl or $C_{1-10}$ alkyl;
----- denotes a single or double bond;
provided that
if X is O, $R_3$ is absent;
if ----- denotes a single bond, the compound has the formula:

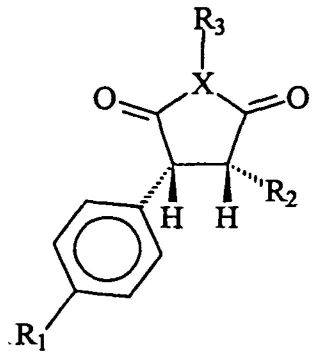

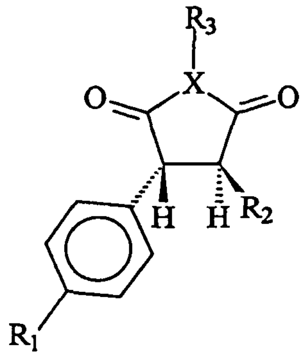

,

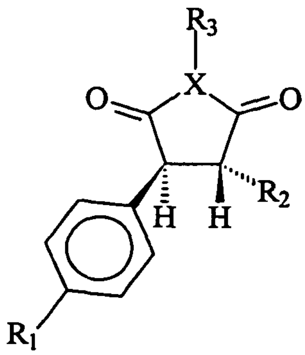

or

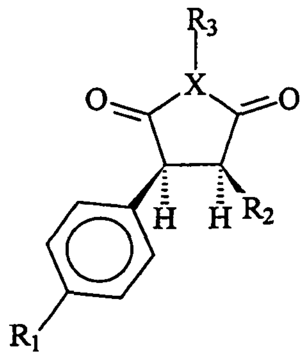

; and

(ii) a pharmaceutically acceptable carrier or diluent, for use in a method of:

(a) decreasing blood pressure;
(b) increasing high density lipoprotein;
(c) central cholinergic agonism;
(d) providing hepatoprotection against D-galactosamine or Hepatitis C virus; or
(e) treating inflammation.

Further object of the present invention is to provide a mycelium of *Antrodia camphorata* for:

(a) decreasing blood pressure;
(b) increasing high density lipoprotein;
(c) central cholinergic agonism;
(d) hepatoprotection against D-galactosamine; Hepatitis C virus; or
(e) treating inflammation,

wherein the mycelium has been previously prepared according to submerged liquid fermentation.

Further object of the present invention is to provide use of a compound as defined in claim 1, or a pharmaceutical composition as defined in claim 6, or a mixture as defined in claim 8, or a mycelium as defined in claim 11, for the manufacture of a medicament for:

(a) decreasing blood pressure;
(b) increasing high density lipoprotein;
(c) central cholinergic agonism;
(d) hepatoprotection against D-galactosamine or Hepatitis C virus; or
(e) treating inflammation.

**BRIEF DESCRIPTION OF THE DRAWINGS**

FIG.1 shows HMBC correlations of compound 2.

FIG2 shows the compounds 1-5 of the present invention.

FIG.3 shows NOE (Nuclear Overhauser Effect) correlations of compounds 4 and 5 of the invention.

FIG.4 (a)-(d) shows test results of compound 3 of the invention.

Fig.5 (a)-(c) shows test results of ACM (*Antrodia camphorata* mycelia powder) $H_2O$ Extract.

FIG.6 (a)-(f) shows test results of ACM EtOH (ethyl alcohol) Extract.

FIG.7 (a)-(e) shows test results of compound 1 of the invention.

FIG.8 shows serum constituents in mice with *P. acnes* plus LPS-induced liver injury. In the figure, N means normal group, C means control group, WE-50 means water extract (50mg/kg), WE-200 means water extract (200mg/kg) and C3 means compound 3 of the invention (20mg/kg).

**DETAILED DESCRIPTION OF THE INVENTION**

The present invention provides a compound having the formula

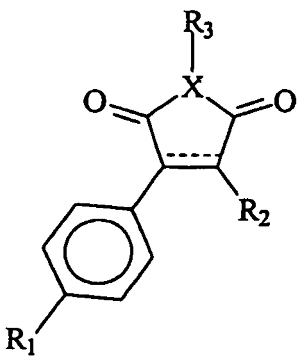

its tautomeric forms, its stereoisomers, its pharmaceutically acceptable salts, or its pharmaceutically acceptable solvates, wherein
X is N or O;
$R_1$ is $C_{1-10}$ alkyloxy, $C_{2-10}$ alkenyloxy, or $C_{2-10}$ alkynyloxy;
$R_2$ is H, $C_{1-10}$ alkyl, $C_{2-10}$ alkenyl or $C_{2-10}$ alkynyl;
$R_3$ is absent, H, hydroxyl or $C_{1-10}$ alkyl;
----- denotes a single or double bond;
provided that
if X is O, $R_3$ is absent;
if ----- denotes a single bond, the compound has the formula:

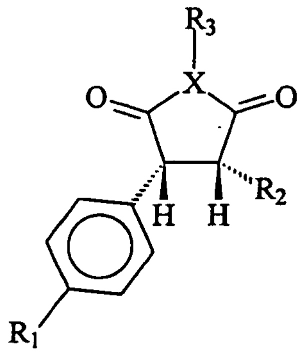

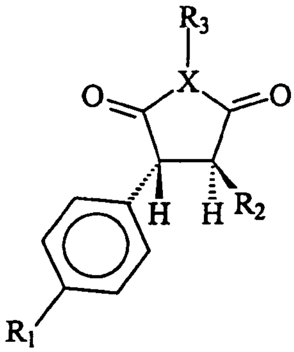

,

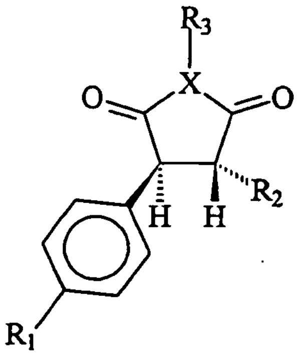

or

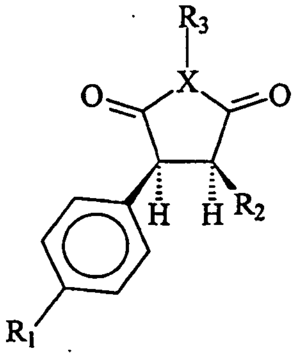

.

for use in a method of:

(a) decreasing blood pressure;
(b) increasing high density lipoprotein;
(c) central cholinergic agonism;
(d) providing hepatoprotection against D-galactosamine or Hepatitis C virus; or
(e) treating inflammation.

In the compound of the invention, the preferred $R_1$ is $C_{2-6}$ alkenyloxy, or $C_{2-6}$ alkynyloxy; the more preferred $R_1$ is $C_{2-6}$ alkenyloxy substituted with $C_{1-6}$ alkyl and the most preferred $R_1$ is butenyloxy substituted with methyl. In the compound of the invention, the preferred $R_2$ is $C_{1-6}$ alkyl, the most preferred $R_2$ is isobutyl.

Accordingly, the preferred compound of the invention is
3-isobutyl-4-[4-(3-methyl-2-butenyloxy)phenyl]furan-2,5-dione,
3-isobutyl-4-[4-(3-methyl-2-butenyloxy)phenyl]-1*H*-pyrrol-2,5-dione,
3-isobutyl-4-[4-(3-methyl-2-butenyloxy)phenyl]-1*H*-pyrrol-1-ol-2,5-dione,
3*R**,4*S**-1-hydroxy-3-isobutyl-4-[4-(3-methyl-2-butenyloxy)phenyl]pyrrolidine-2,5-dion e, or
3*R**,4*R**-1-hydroxy-3-isobutyl-4-[4-(3-methyl-2-butenyloxy)phenyl]pyrrolidine-2,5-dion e.

The further preferred compound of the invention is
3-isobutyl-4-[4-(3-methyl-2-butenyloxy)phenyl]-1*H*-pyrrol-2,5-dione or
3-isobutyl-4-[4-(3-methyl-2-butenyloxy)phenyl]-1*H*-pyrrol-1-ol-2,5-dione. The further preferred compound of the invention is
3-isobutyl-4-[4-(3-methyl-2-butenyloxy)phenyl]-1*H*-pyrrol-1-ol-2,5-dione.

Certain compounds may exist in one or more particular geometric, optical, enantiomeric, diasteriomeric, epimeric, stereoisomeric, tautomeric, conformational, or anomeric forms, including but not limited to, cis- and trans-forms; E- and Z-forms; c-, t-, and r-forms; endo- and exo-forms; R-, S-, and meso-forms; D- and L-forms; d- and 1-forms; (+) and (-) forms; keto-, enol-, and enolate-forms; syn- and anti-forms; synclinal- and anticlinal-forms; .alpha.- and .beta.-forms; axial and equatorial forms; boat-, chair-, twist-, envelope- and halfchair-forms; and combinations thereof, hereinafter collectively referred to as "isomers" (or "isomeric forms").

If the compound is in crystalline form, it may exist in a number of different polymorphic forms.

The above exclusion does not pertain to tautomeric forms, for example, keto-, enol-, and enolate-forms, as in, for example, the following tautomeric pairs: keto/enol, imine/enamine, amide/imino alcohol, amidine/amidine, nitroso/oxime, thioketone/enethiol, N-nitroso/hyroxyazo, and nitro/aci-nitro.

Unless otherwise specified, the compounds of the present invention include all such isomeric forms, including (wholly or partially) racemic and other mixtures thereof. Methods for the preparation (e.g. asymmetric synthesis) and separation (e.g. fractional crystallization and chromatographic means) of such isomeric forms are either known in the art or are readily obtained by adapting the methods taught herein, or known methods, in a known manner.

Unless otherwise specified, the compounds of the present invention include also includes ionic, salt, solvate, for example, as discussed below.

It may be convenient or desirable to prepare, purify, and/or handle a corresponding salt of the active compound, for example, a pharmaceutically-acceptable salt. Examples of pharmaceutically acceptable salts are discussed in Berge et al., 1977, "Pharmaceutically Acceptable Salts," J. Pharm. Sci., Vol. 66, pp. 1-19.

The compound of the invention may be in isolated form in which case it will generally comprise at least 80% e.g. 90, 95, 97 or 99% of the dry mass in the preparation. The compound of the invention may be free of other cellular components.

The present invention also provides a mixture from mycelium of *Antrodia camphorata* for:

(a) decreasing blood pressure;
(b) increasing high density lipoprotein;
(c) central cholinergic agonism;
(d) hepatoprotection against D-galactosamine; Hepatitis C virus; or
(e) treating inflammation,

wherein the mycelium has been previously prepared according to submerged liquid fermentation. The mixture of the invention is prepared from water or organic solvent extract of mycelium of *Antrodia camphorata.* The organic solvent includes but is not limited to alcohol (such as $CH_3OH$, $C_2H_5OH$, $C_3H_7OH$), ester (such as acetyl acetate), alkane (such as hexane) and halogenated alkane (such as $CH_3Cl$, $C_2H_2Cl_2$). The preferred organic solvent is ethanol or alcoholic solvent without causing any side effect of human. The mixture of the invention can decrease blood pressure or increase high density lipoprotein. In addition, the same mixture has central cholinergic agonism (such as the use of treating or preventing Alzheimer's diseases, dementia, etc., Lisa R. Fodero, David H. Small *"Cholinergic abnormalities in Alzheimer's disease: are there new targets for drug development?" Drug Development Research, Vol. 56, Issue 3, pp 369-379, 2002), hepatoprotection, anti-inflammation or anti-tumor activity. Especially, the same mixture can be applied to treat hepatitis caused by chemical compounds (such as $CCl_4$ etc.) or biological substance (such as microorganisms HBV, HCV etc.).

The mixture of the invention can inhibit tumor from the cells or tissues selected from the group consisting of liver, intestine, bone, blood, lymph and breast. The subject accepting the mixture of the invention includes but is not limited to human, mammal, mouse, rat, horse, pig, chicken, duck, dog and cat.

Optionally the compound of the invention may be provided in isolated form for inclusion in a mixture or composition. The composition may be a pharmaceutical composition. Such a pharmaceutical composition may comprise a pharmaceutically acceptable carrier or diluent.

The present invention also provides a pharmaceutical composition comprising:

(i) a compound having the formula

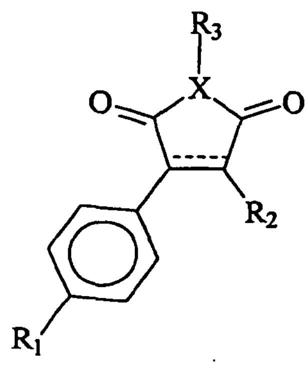

its tautomeric forms, its stereoisomers, its pharmaceutically acceptable salts, or its pharmaceutically acceptable solvates,
wherein
X is N or O;

$R_1$ is $C_{1-10}$ alkyloxy, $C_{2-10}$ alkenyloxy, or $C_{2-10}$ alkynyloxy;
$R_2$ is H, $C_{1-10}$ alkyl, $C_{2-10}$ alkenyl or $C_{2-10}$ alkynyl;
$R_3$ is absent, H, hydroxyl or $C_{1-10}$ alkyl;
----- denotes a single or double bond;
provided that
if X is O, $R_3$ is absent;
if ----- denotes a single bond, the compound has the formula:

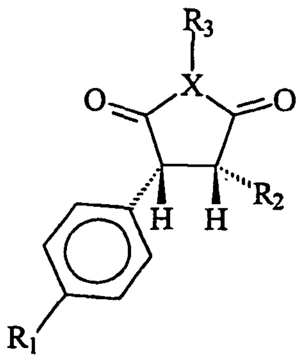

,

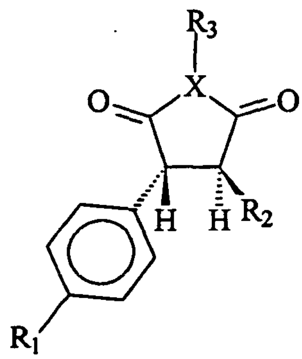

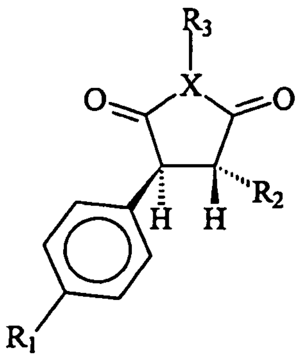

or

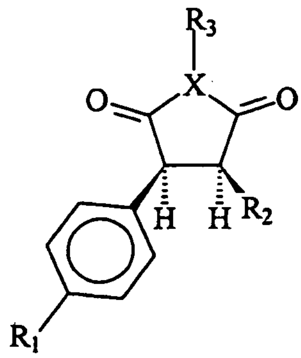

; and
(iii) a pharmaceutically acceptable carrier or diluent, for use in a method of:

(a) decreasing blood pressure;
(b) increasing high density lipoprotein;
(c) central cholinergic agonism;
(d) providing hepatoprotection against D-galactosamine or Hepatitis C virus; or
(e) treating inflammation.

The composition of the invention can decrease blood pressure or increase high density lipoprotein. In addition, the composition of the invention has central cholinergic agonism, hepatoprotection, anti-inflammation or anti-tumor activity. Especially, the composition of the invention can inhibit tumor from the cells or tissues selected from the group consisting of liver, intestine, bone, blood, lymph and breast. The subject accepting the composition of the invention includes but is not limited to human, mammal, mouse, rat, horse, pig, chicken, duck, dog and cat.
The present invention also provides a novel mycelium of *Antrodia camphorate* for:

(a) decreasing blood pressure;
(b) increasing high density lipoprotein;
(c) central cholinergic agonism;

(d) hepatoprotection against D-galactosamine; Hepatitis C virus; or
(e) treating inflammation,

wherein the mycelium has been previously prepared according to submerged liquid fermentation. The preferred mycelium has at least 1% of the weight of raw mycelium being the total weight of the compounds 1-5 of the invention. The most preferred mycelium has at least 3% of the weight of raw mycelium being the total weight of the compounds 1-5 of the invention. The mycelium of *Antrodia camphorata* is previously prepared according to submerged liquid fermentation such as T. L. M. Stamford et al., Food Science "Protein enrichment of cashew wastes for animal feeds" at http://www.unu.edu/unupress/food/8F101e/8F101E0b.htm.

## EXAMPLES

The examples below are non-limiting and are merely representative of various aspects and features of the present invention.

General Experimental Procedures

Melting points were measured on a Yanagimoto micro hot-stage melting point apparatus and uncorrected. Optical rotations were measured with a Jasco DIP-360 automatic polarimeter. UV spectra were measured with a Shimadzu UV-2200 recording spectrophotometer. IR spectra were measured with a Jasco FT/IR-230 infrared spectrometer. $^{1}H$- and $^{13}C$-NMR spectrum were measured with a Varian Unity Plus 500 spectrometer. EIMS and HR-EIMS were measured with a Jeol JMS-AX 505 HAD mass spectrometer at an ionization voltage of 70 eV Column chromatography was carried out on silica gel BW-820MH (normal phase) and Chromatorex-ODS DM1020T (reversed phase) (Fuji Silysia).

Extraction and Isolation

*Antrodia camphorata* mycelia powder (ACM) (60 g), from Simpson Biotech Co. Ltd., Taiwan, October 2001, were three times extracted with $CHCl_3$ for 3 h under reflux. The $CHCl_3$ extract (5.3 g) was chromatographed on silica gel eluted with *n*-hexane-acetone (19:1-14:6), and $CHCl_3$-MeOH (1:1) to give nine fractions (Fr. 1-9). Fraction 2 was chromatographed on silica gel to give compound **1** (8.7 mg). Fraction **4** was chromatographed on normal and reversed phase silica gel to give compound **2** (13.6 mg). Fraction 5 was chromatographed on silica gel eluted with *n*-hexane-acetone (8: 2) to give ergosterol peroxide (35.8 mg). Fraction 6 gave compound **3** (14.6 mg) by combination of normal and reversed phase silica gel column chromatography. Fraction 7 yielded a mixture of compounds **4** and 5 (4:1) by column chromatography. The mixture of compounds **4** and **5** were subsequently separated by preparative HPLC [column: Tosoh TSK-gel ODS-80$T_M$ (21.5 x 300 mm), mobile phase: $CH_3OH$-$H_2O$ containing 0.1% TFA (70:30)].

**3-Isobutyl-4-[4-(3-methyl-2-butenyloxy)phenyl]furan-2,5-dione (compound** 1):

yellow oil; UV (MeOH) $\lambda_{max}$ (log ε) 227 (4.1), 258 (3.9), 275 (3.8), 355 (3.4) nm; IR ($CHCl_3$) $v_{max}$ 1763 $cm^{-1}$ ; $^{1}H$-NMR Table 1; $^{13}C$-NMR Table 2; EIMS *m*/*z* 314 $[M]^+$ (100), 246 (100), 131 (100); HR-EIMS *m*/*z* 314.1523 (Calcd for $C_{19}H_{22}O_4$, 314.1518).

3-Isobutyl-4-[4-(3-methyl-2-butenyloxy)phenyl]-1*H*-pyrrole-2,5-dione **(2):**

yellow needles (*n*-hexane-AcOEt); mp 110-111 °C; UV (MeOH) $\lambda_{max}$ (log ε) 230 (4.3), 272 (3.5), 355 (3.7) nm; IR ($CHCl_3$) $V_{max}$ 1724 $cm^{-1}$; $^{1}H$-NMR Table 1; $^{13}C$-NMR Table 2; EIMS *m*/*z* 313 $[M]^+$ (8), 245 (100), 203 (77), 131 (28); HR-EIMS *m*/*z* 313.1681 (Calcd for $C_{19}H_{23}NO_3$, 313.1678).

**X-ray Crystallography of Compound 2:**

Yellow needles were obtained by crystallization from *n*-hexane-AcOEt and selected for data collection. Crystal data: $C_{19}H_{23}NO_3$; $M_r$=313.40; dimensions 0.15 x 0.02 x 0.02 mm; triclinic, space group P1 (#2), a=6.3505(5) Å, b=12.205 (1) Å, c=12.560(2) Å, $\alpha$=64.623(7)°, $\beta$=75.358(4)°, $\gamma$=84.681(5)°, V=850.9(2) $Å^3$, *Z*=2, $D_{calc}$=1.223 g/$cm^3$, $\mu$(MoK$\alpha$)=0.82 $cm^{-1}$, $F_{000}$=336.00. Measurement was made on a Rigaku RAXIS-RAPID Imaging Plate diffractometer with graphite monochromated Mo-K$\alpha$ ($\lambda$=0.71069 Å) radiation at 93 K.
Of the 8950 reflections that were collected, 4745 were unique ($R_{int}$=0.108); equivalent reflections were merged. The crystal structure was solved by direct methods (SHELXS86) and refined by full-matrix least-squares. The non-hydrogen atoms were refined anisotropically. Hydrogen atoms were included but not refined. The final indices were *R*=0.074, $R_w$=0.099, with GOF (Guest Observer Facility) =1.06. The maximum and minimum peaks on the final difference Fourier map corresponded to 0.83 and -0.89 $e^-/Å^3$, respectively.

**3-Isobutyl-4-[4-(3-methyl-2-butenyloxy)phenyl]-1*H*-pyrrol-1-ol-2,5-dione (compound 3):**

yellow oil; UV (MeOH) $\lambda_{max}$ (log ε): 232.5 (4.3), 296 (3.7), 374 (3.7) nm; IR ($CHCl_3$) $v_{max}$ 1717 $cm^{-1}$ ; $^1$H-NMR Table 1; $^{13}$C-NMR Table 2; EIMS *m*/*z* 329 $[M]^+$ (12), 261 (100), 131 (50); HR-EIMS *m*/*z*: 329.1637 (Calcd for $C_{19}H_{23}NO_4$, 329.1627).

***3R**,*4S**-1-Hydroxy-3-isobutyl-4-[4-(3-methyl-2-butenyloxy)phenyl] pyrrolidine-2,5-dione (4):**

colorless oil; $[\alpha]_D^{23}$ +2.5° (c 0.2, MeOH); UV (MeOH) $\lambda_{max}$ (log ε): 225 (4.3), 275 (3.3), 283 (3.2) nm; IR ($CHCl_3$) $v_{max}$ 1715 $cm^{-1}$; $^1$H-NMR Table 1; $^{13}$C-NMR Table 2; EIMS *m*/*z* 331 $[M]^+$ (2), 263 (67), 207 (66), 191 (30), 179 (40), 133 (64), 69 (100); HR-EIMS *mlz* 331.1747 (Calcd for $C_{19}H_{25}NO_4$, 331.1783).

*3R*,4R**-1-Hydroxy-3-isobutyl-4-[4-(3-methyl-2-butenyloxy)phenyl] **pyrrolidine-2,5-dione (5):**

colorless oil; $[\alpha]D^{23}$ +3.0° (c 0.2, MeOH); UV (MeOH) $\lambda_{max}$ (log ε): 227 (4.3), 275 (3.4), 283 (3.3) nm; IR ($CHCl_3$) $v_{max}$ 1715 $cm^{-1}$; $^1$H-NMR Table 1; $^{13}$C-NMR Table 2; EIMS *m*/*z* 331 $[M]^+$ (1), 263 (45), 207 (50), 191 (75), 179 (30), 133 (100), 69 (92); HR-EIMS *m*/*z* 331.1766 (Calcd for $C_{19}H_{25}NO_4$, 331.1783).

**Ergosterol peroxide:**

colorless needles (*n*-hexane-acetone); mp 165-169 °C (lit[2] mp 171-174°C).

**Cytotoxic Assays.**

*The in vitro* LLC tumor cell assay was carried out by sulforhodamin B (SRB) method. The 50% growth inhibition ($ED_{50}$) was calculated by Probit method.

Results and Discussion

The $CHCl_3$ extract of the mycelium of *Antrodia camphorata* was repeatedly chromatographed on normal and reversed phase silica gel to afford five new maleic and succinic acid derivatives (compounds **1-5**) together with ergosterol peroxide.

Table 1

Table 1. $^1$H-NMR Spectral Data of Compounds **1-5** (δppm, *J*=Hz) (500 MHz, $CDCl_3$)

| H | **1** | **2** | **3** | **4** | **5** |
|---|---|---|---|---|---|
| 3 | - | - | - | 2.87 (1H, m) | 3.08 (1H, m) |
| 4 | - | - | - | 3.52 (1H, d, *J*=4.0) | 4.07 (1H, d, *J*=8.0) |
| 1' | 2.59 (2H, d, *J*=7.0) | 2.51 (2H, d, *J*=7.0) | 2.50 (2H, d, *J*=7.0) | 1.51 (1H, m)<br>1.72~1,84 (1H) | 1.02 (1H, m)<br>1.42~1.48 (1H) |
| 2' | 2.12 (1H, sep, *J*=7.0) | 2.06 (1H, sep, *J*=7.0) | 2.05 (1H, sep, *J*=7.0) | 1.72~1.84 (1H) | 1.42~1.48 (1H) |
| 3'<br>4' | 0.94 (6H, d, *J*=7.0) | 0.90 (6H, d, *J*=7.0) | 0.88 (6H, d, *J*=7.0) | 0.70 (3H, d, *J*=6.5)<br>0.89 (3H, d, *J*=6.5) | 0.66 (3H, d, *J*=6.5)<br>0.80 (3H, d, *J*=6.5) |
| 2", 6" | 7.50 (2H, d, *J*=9.0) | 7.50 (2H, d, *J*=9.0) | 7.50 (2H, d, *J*=9.0) | 7.07 (2H, d, *J*=8.5) | 6.96 (2H, d, *J*=9.0) |
| 3", 5" | 7.02 (2H, d, *J*=9.0) | 6.95 (2H, d, *J*=9.0) | 6.98 (2H, d, *J*=9.0) | 6.87 (2H, d, *J*=8.5) | 6.84 (2H, d, *J*=9.0) |
| 1'" | 4.57 (2H, d, *J*=6.6) | 4.56 (2H, d, *J*=6.5) | 4.55 (2H, d, *J*=6.9) | 4.47 (2H, d, *J*=6.5) | 4.47 (2H, d, *J*=6.5) |

(continued)

| Table 1. $^1$H-NMR Spectral Data of Compounds **1-5** (δppm, *J*=Hz) (500 MHz, $CDCl_3$) | | | | | |
|---|---|---|---|---|---|
| H | **1** | **2** | **3** | **4** | **5** |
| 2''' | 5.50 (1H, brt, *J*=6.6) | 5.50 (1H, brt, *J*=6.5) | 5.49 (1H, brt, *J*=6.9) | 5.47 (1H,brt, *J*=6.5) | 5.47 (1H,brt, J=6.5) |
| 4''' | 1.81 (3H, s) | 1.81 (3H, s) | 1.81 (3H, s) | 1.79 (3H, s) | 1.79 (3H, s) |
| 5''' | 1.76 (3H, s) | 1.76 (3H, s) | 1.76 (3H, s) | 1.73 (3H, s) | 1.73 (3H, s) |

Table 2.

| $^{13}$C-NMR Spectral Data for Compound **1-5** (δ ppm) (125 MHz, $CDCl_3$) | | | | | |
|---|---|---|---|---|---|
| C | **1** | **2** | **3** | **4** | **5** |
| 2 | 166.4 (s) | 171.7 (s) | 168.8 (s) | 174.8 (s) | 175.1 (s) |
| 3 | 139.8 (s) | 138.8 (s)[a)] | 135.9 (s)[a)] | 44.6 (d) | 40.3 (d) |
| 4 | 140.2 (s) | 139.2 (s)[a)] | 136.0 (s)[a)] | 49.8 (d) | 47.5 (d) |
| 5 | 165.4 (s) | 171.1 (s) | 168.1 (s) | 173.2 (s) | 173.6 (s) |
| 1' | 33.6 (t) | 32.8 (t) | 33.2 (t) | 40.4 (t) | 35.3 (t) |
| 2' | 27.9 (d) | 28.1 (d) | 28.4 (d) | 25.3 (d) | 25.2 (d) |
| 3' | 22.7 (q) | 22.7 (q) | 23.0(q) | 21.3 (q) | 21.8 (q) |
| 4' | | | | 23.0 (q) | 22.4 (q) |
| 1 " | 119.9 (s) | 121.2 (s) | 120.8 (s) | 127.9 (s) | 125.1 (s) |
| 2", 6" | 131.1 (d) | 130.9 (d) | 131.0 (d) | 128.8 (d) | 130.2 (d) |
| 3",5" | 115.1 (d) | 114.9 (d) | 115.0 (d) | 115.4 (d) | 115.0 (d) |
| 4" | 160.9 (s) | 160.1 (s) | 160.2 (s) | 158.7 (s) | 158.7 (s) |
| 1''' | 65.0 (t) | 64.9 (t) | 65.1 (t) | 64.1 (t) | 64.8 (t) |
| 2''' | 118.7 (d) | 119.3 (d) | 119.2 (d) | 119.4 (d) | 119.3 (d) |
| 3''' | 139.1 (s) | 138.6 (s)[a)] | 138.9 (s) | 138.3 (s) | 138.4 (s) |
| 4''' | 25.2 (q) | 25.8 (q) | 26.1 (q) | 25.8 (q) | 25.8 (q) |
| 5''' | 18.2 (q) | 18.2 (q) | 18.5 (q) | 18.1 (q) | 18.2 (q) |
| a) Assignments may be interchangeable. | | | | | |

The structures of the new compounds were determined as follow:

The molecular formula of compound **1** was assigned as $C_{19}H_{22}O_4$ by HR-EIMS. The IR spectrum revealed carbonyl absorption of acid anhydride at 1763 $cm^{-1}$. The $^1$H-NMR spectrum of compound **1** was similar to that of compound **2,** and showed the presence of an isobutyl moiety, a 3-methyl-2-butenyloxy moiety, and a *para*-substituted benzene ring. From the HMBC spectrum, compound **1** was demonstrated to have the same partial structure to compound **2** (Figure 1), in which the presence of a maleic anhydride group was deduced on the basis of the molecular formula compound **1** was consequently determined as 3-isobutyl-4-[4-(3-methyl-2-butenyloxy)phenyl]furan-2,5-dione.

Compound **2** gave yellow needles, mp 110-111°C, and the molecular formula $C_{19}H_{23}NO_3$ was assigned by HR-EIMS. The IR spectrum showed an imide carbonyl absorption at 1724 $cm^{-1}$. The $^{13}$C-NMR spectrum showed signals of four methyl carbons, two methylene carbons, and one methine carbon in the aliphatic region, as well as one benzene ring, one olefinic group and two carbonyl carbons. The $^1$H-NMR spectrum showed the presence of an isobutyl moiety at δ 0.90, 2.06, and 2.51, a 3-methyl-2-butenyloxy moiety at δ 1.76, 1.81, 4.56, and 5.50, and a *para*-substituted benzene moiety at δ 6.95 and 7.50, which was further supported by $^1$H-$^1$H COSY (cooler synchrotron) and HMQC (heteronuclear multiple quantum coherence) experiments. Long-range correlations were observed by HMBC as shown in Figure 1. On the basis of the molecular formula and the C-NMR spectrum, this compound was deduced to contain further CHNO atoms, including one more carbonyl carbon. Thus, this ambiguous part was speculated to be a maleimide group. This structure was then established to be 3-isobutyl-4-[4-(3-methyl-2-butenyloxy)phenyl]-1*H*-pyrrole-2,5-dione by X-ray analysis.

The molecular formula of compound **3** was assigned as $C_{19}H_{23}NO_4$ by HR-EIMS. The IR spectrum showed

carbonyl absorption at 1717 $cm^{-1}$, assignable to a hydroxy imide. The $^{1}H$- and $^{13}C$-NMR spectra were also similar to those of compounds **1** and **2,** and showed the presence of an isobutyl moiety, a 3-methyl-2-butenyloxy moiety, and a *para*-substituted benzene ring. In the HMBC experiment, compound **3** was shown to have the same partial structure as compound **2** (Figure 1). Compound **3** contains one more oxygen atom than compound **2,** therefore, this compound was determined to be (3-isobutyl-4-[4-(3-methyl-2-butenyloxy)phenyl-1*H*-pyrrol-1-ol-2,5-dione.

Compounds **4** and **5** had the same $R_f$ values and the same molecular formula by HR-EIMS ($C_{19}H_{25}NO_4$, found 331.1747 and 331.1766, respectively), however, they could be separated by preparative HPLC. The IR spectrum of both compounds showed a hydroxy imide carbonyl absorption at 1715 $cm^{-1}$. In the $^{1}H$- and $^{13}C$-NMR spectra, both compounds showed the presence of an isobutyl moiety, a 3-metyl-2-butenyloxy moiety, and a *para*-substituted benzene ring, but the isobutyl methylene protons displayed a multiplet and not a doublet as for compounds **1-3.** The $^{1}H$-$^{1}H$ COSY spectrum indicated that this methylene group is attached to a -CH-CH- unit. The $^{13}C$-NMR spectra of compounds **4** and **5** exhibited two additional $sp^3$ carbon signals, replacing two $sp^2$ carbon signals observed for compounds **1-3.** Therefore, compounds **4** and **5** were not *N*-hydroxy maleimides, but rather *N*-hydroxy succinimides, with stereocenters at positions C-3 and C-4 in the succinimide ring. Compounds **4** and **5** were determined to be *trans* and *cis* isomers, respectively, from the coupling constant between H-3 and H-4 (4.0 and 8.0 Hz for compounds **4** and 5, respectively). No NOE was observed between H-3 and H-4 in the NOESY (Nuclear Overhauser Effect Spectroscopy) spectrum of compound **4,** while appreciable NOE was observed in that of compound **5.** The optical rotations of compounds **4** and **5** showed +2.5° and +3.0°, respectively, while their CD spectra showed no Cotton effects at any wave length, suggesting that both compounds **4** and **5** are racemic mixtures. Resolution of these racemic mixtures by HPLC using a chiral column with several solvent systems was unsuccessful. At present, we cannot definitely conclude whether these compounds are optically active compounds or racemic mixtures. Thus, their relative structures were determined as *3R*,4S**- and *3R *,4R**-1-hydroxy-3-isobutyl-4-[4-(3-methyl-2-butenyloxy)phenyl]pyrrolidine-2, 5-dione, respectively.

The cytotoxic activity of the chloroform extract and isolated compounds were investigated using LLC (Lewis lung carcinoma) cell line (Table 3). The chloroform extract showed moderate cytotoxic effects with an 50% Growth Inhibition ($ED_{50}$) value of 26.7 μg/ml. Maleic compounds **1** and **4** had no cytotoxic activity, whereas compounds **2** and **3** were found to be cytotoxic to the LLC cell line with $ED_{50}$ values lower than that of the chloroform extract.

Table 3. $ED_{50}$ of the $CHCl_3$ Extract and Compounds **1-4** from the mycelia of *Antrodia camphorata* against LLC Cell Line.

| | $ED_{50}$ (μg/ml) |
|---|---|
| $CHCl_3$ extract | 26.7 |
| **1** | >20 |
| **2** | 3.6 |
| **3** | 7.5 |
| **4** | >10 |
| Adriamycin[a)] | 0.14 |

a) Positive control.

**Tumor assay of ACM (*Antrodia camphorata* mycelia powder)**

A. Cell line

Adherent cell:

MCF-7: human breast carcinoma
HT-29: human colon adenocarcinoma
KATO III: human stomach carcinoma
SW480: human colon adenocarcinoma
SW620: human colon adenocarcinoma
HepG2: human liver carcinoma

Suspension cell:

EL4: mice lymphoma

B. Samples
Compound 1, Compound 3, ACM EtOH Extract, ACM $H_2O$ Extract

C. Assay method
Calculate $ED_{50}$(50% inhibition of effective dose)
Adherent cell: MTT (methyl thiazolyl tetrazolium) method; for MCF-7, HT-29, KATO III, SW480, HepG2, cells are determined at 3 days. SW620 at 4 days
Suspension cell: Cell count method: EL4 cells count at 5 days

D. Result
Calculation: y = m Ln(x) + b

Example

| X | Y |
|---|---|
| 0 | 0.97 |
| 10 ppm | 0.941 |
| 30 ppm | 0.6 |
| 100 ppm | 0.331 |

Use value of X (10, 30, 50 ppm) and Y to get correlation curve y = -0.2643Ln(x) + 1.5321

$$ED_{50} = \exp[(0.97/2-1.5321)/(-0.2643)]$$

Sample preparation and sample description

A. ACM (*Antrodia camphorata* mycelia powder) $H_2O$ Extract

1. Add 1 g of ACM into 40 ml of RO $H_2O$ in a 250 ml beaker, put the beaker in ultrasonic water bath for 20 min at room temperature
2. Stir at 45°C water bath for 45 min
3. Place the beaker in ultrasonic water bath for another 20 min
4. Centrifuge the sample at 3000 rpm for 15 min
5. Collect supernatant and perform serial dilutions with media.

B. Determination of sample concentration

1. Weigh an evaporating dish (W1)
2. Add 10 ml of $H_2O$ extract sample in the evaporating dish
3. Place the evaporating dish in the oven to remove water (W2) Sample weight/ml = (W2-W1)/10

C. ACM (*Antrodia camphorata* mycelia powder) EtOH Extract

1. Add 100 ml of 95□alcohol to 20 g ACM in a 500 ml beaker and stir for 10 min at room temperature
2. Filter the suspension through Advantec #1 filter paper, and collect the filtrate
3. Concentrate the filtrate by rotary vacuum evaporator to remove alcohol.

D. Compound **1**: Pure compound from ACM

E. Compound **3**: Pure compound from ACM

MTT assay method

1. Discard old media after cell proliferation, then wash cells once with phosphate-buffered saline (PBS) once
2. Wash down the cells with trypsin-EDTA
3. Centrifuge at 1200 rpm for 5 min, then discard supernatant
4. Suspend the pellet with 10 ml medium
5. Mix 100 μl cell suspension with 100 μl trypan blue to calculate viable cells
6. Add 1 x $10^4$ cells/100 μl medium in each well of the 96 well plate, incubate the plate $CO_2$ incubator at 37°C for 24 hrs
7. Discard old medium, wash cells once with PBS
8. Add 100μl sample in each well, incubate the plate in $CO_2$ incubator at 37°C
9. Wash cells once with PBS at 3rd, 4th and 5th days,
10. Add 57 μl MTT (0.88 mg/ml) in each well
11. After 4 hrs discard MTT and wash cells with PBS once
12. Add 50 μl DMSO /Well
13. Read at OD545 on Elisa reader

Cell count method (EL4 cell line)

1. Discard old media after cell proliferation by centrifugation
2. Resuspend the pellet with fresh medium
3. Mix 100 μl cell suspension with 100 μl trypan blue to calculate viable cells
4. Prepare different concentration of samples that contain 1*$10^5$ cell/ml sample
5. Load 100 μl sample in each well of the 96 well plate, incubate the plate at 37°C $CO_2$ incubator
6. Calculate viable cells at 3rd, 4th and 5th days

PBS

| | |
|---|---|
| NaCl | 8g |
| KCl | 0.2g |
| $Na_2HPO_4$ | 1.4g |
| $KH_2PO_4$ | 0.2g |
| Make volume to 1L | PH 7.4 |

Result and Discussion

Table 4. $ED_{50}$ of ACM on Cell Lines

| Cell line | HepG2 | HT-29 | KATO III | EL4 | SW480 | SW620 | MCF-7 |
|---|---|---|---|---|---|---|---|
| Compound **1** | 21 ppm | 52 ppm | 38 ppm | 3.5 ppm | | 15 ppm | 6 ppm |
| Compound **3** | 35 ppm | 42 ppm | 69 ppm | 2.6 ppm | 20 ppm | 27 ppm | 0.02 ppm |
| ACM EtOH Extract | 32 ppm | 52 ppm | 156 ppm | 2.6 ppm | 71 ppm | 4 ppm | |
| ACM $H_2O$ Extract | 295 ppm | 707 ppm | | 20 ppm | 207 ppm | 132 ppm | 318 ppm |

Detailed test results as follows:

Compound 3 of the invention: HepG2 (Fig. 4a), EL4 (Fig. 4b), HT-29(Fig. 4c) and Kato III (Fig. 4d).

ACM $H_2O$ Extract: HepG2 (Fig. 5a), SW620 (Fig. 5b) and EL4 (Fig. 5c).
ACM EtOH Extract: HT-29(Fig. 6a), SW480 (Fig. 6b), SW620 (Fig. 6c), EL4 (Fig. 6d), HepG2 (Fig. 6e) and Kato III (Fig. 6f).
Compound 1 of the invention: MCF-7(Fig. 7a), EL4 (Fig. 7b), HT-29(Fig. 7c), SW620 (Fig. 7d) and HepG2 (Fig. 7e).

Given the above the compounds and ACM Extract of the invention have inhibition effect on various types of tumor cells.

**Analysis of all new compounds (1, 2 AND 3) from ACM EtOH extract by high performance of liquid chromatography method**

Purpose: In order to measure the amount of all new compounds (**1**, **2** and **3**) from ACM EtOH Extract, High Performance of Liquid Chromatography (HPLC) was employed as our routine quality control procedures.
Preparation for ACM EtOH Extract sample:

1. By using digital balance precisely weight 20.000(g) of sample powders in a graduated media lab bottle with 100 mL of 95% alcohol, and do not screw the lid tightly on.
2. Place above step of the sample bottle in ultrasonic water bath 10 minutes.
3. Pour liquid samples to centrifuge tubes, and then place those samples in a centrifuge remove crude particle, under condition of 6500 rpm/ 5 minutes.
4. Filter liquid layer with filter paper, advantec No. 1.
5. Concentrate filtering solution by rotary vacuum evapotator until appear a thick, yellowish liquid, alcohol free.
6. Repeat three times of step 1 to 5, and then collect all extract product (total ACM EtOH Extract = 4.60 g). Calculate yield.

**Application By Water HPLC, Model 2690:**

1. Column: Reverse Phase C 18
2. Mobile Phase: MeOH·$H_2O$·acetonitrile
3. Injection vol: 20 μL
4. Detection: Photodiode Array Detector 996 on wavelength 254 nm
5. Preparation 1.000 (g) ACM EtOH Extract sample in 10 mL of alcohol for HPLC analysis*:

**Results**: according to HPLC analysis, the extract product contains pure compound **1**, **2**, and **3** was showed in following Table 5 Table 5:

| For ingredients of three standard compounds :weight 0.0100 (g) in 1 mL of alcohol | | | |
|---|---|---|---|
| Standard Name | Compound **1** | Compound **2** | Compound **3** |
| Concentration (g/mL) | 0.01 | 0.01 | 0.01 |
| Peak Area | 49,315,783 | 129,327,136 | 136,255,406 |
| Retention time (min) | 134.8 | 124.3 | 119.8 |
| *For ACM EtOH Extract sample :weight 1.000 (g) in 10 mL of alcohol | | | |
| Concentration (g/mL) | $8.59 \times 10^{-3}$ | $5.59 \times 10^{-4}$ | $1.659 \times 10^{-2}$ |
| Peak Area | 42,374,766 | 7,226,937 | 226,102,223 |
| Percentage yield % (w/w) | 8.59 | 0.559 | 16.59 |

Therefore, the total weight of compounds 1, 2 and 3 is 5.92% by weight in ACM sample.

**Tests for ACM-EtOH Extract**

Materials and Equipment

1. Test Substances and Dosing Pattern

Test substance was administered orally at an initial dose of 1000 mg/kg for all *in vivo* assays in a vehicle of 2% Tween 80. Time of observation for each assay was described in methods.

2. Animals

Male or female ICR mice, Wistar-Okamoto derived male spontaneously hypotensive rat (SHR), Wistar and Long Evans derived rats provided by MDS Pharma Services Taiwan Ltd. were used. Space allocation for animals was as follows: 29 x 18 x 13 cm for 10 mice, 45 x 23 x 21 cm for 6 rats, and 45 x 23 x 21 cm for 3 guinea pigs. Mice and rats were housed in $APEC^R$ cages. The immunocompetent C57BL/6J male mice, 6-8 weeks age, weighing $21\pm2$ gm were also used in this study and provided by National Taiwan University Animal Center. The animals were housed in Individually Ventilated Cages Racks (IVC racks, 36 Mini Isolator System). Each cage was sterilized by autoclave and contained 5 mice (in 26.7x20.7x14 cm). All animals were maintained in a controlled temperature (21 ° -23 °C) and humidity (60%-70%) environment with 12 hour light dark cycles for at least one week in the laboratory prior to use. Free access to standard lab chow (LabDiet Rodent Diet and Guinea Pig Diet, PMI Nutrition International, USA) and tap water was granted.

3. Cell line and Culture Media

The murine melanoma cell line, B16-F0 (ATCC CRL-6322), was purchased from American Type Culture Collection and Dulbecco's Modified Eagle's Medium (GIBCO, USA) was used as culture medium. The tumor cells were incubated in an atmosphere containing 5% $CO_2$ at 37°C.

4. Chemicals

General:

Distilled Water (In-house), Dimethyl Sulfoxide (DMSO, Merck, Germany), Isotonic Sodium Chloride Solution (Sintong Chemical Industry Co. Ltd., R.O.C.), magnesium Sulfate ($MgSO_4.7H_20$, Wako, Japan), Meclofenamate Sodium (Sigma, USA), Methylcellulose (Signa, USA), Sodium Hydroxide (NaOH, Wako, Japan), Phosphate Buffered Saline (Sigma, USA) and Tween 80 (Wako, Japan).

Reagents

Glicose-HA assay kit (Wako, Japan), Alanine aminotransferase (ALT) assay kit (Wako, Japan), Aspartate aminotransferase (AST) assay kit (Wako, Japan), T-Cholesterol-HA and HDL assay kit (Wako, Japan), Hemolynac 3 Hemolys (Nihon Koden, Japan), Isotonic 3 Diluent (Nihon Koden, Japan).

5. Equipment

General Use:

Animal Case (ShinTeh, R.O.C.), Beaker 250 ml and 1000 ml (Kinmax, USA), disposable syringe (1ml, Top Corporation, Japan), Forceps stainless (klappencker, Germany), Mouse scale #Z-40 (Taconic, USA), needle for oral administration (Natsune, Japan), Needle Hypodermic 23 G x 1" (Top Corporation, Japan), pH Meter (Suntex, USA), Rat scale 500g $\pm2$ g (Chien-chun, ROC), syringe Glass 1 ml, 2 ml and 5 ml (Mitsuba, Japan), and Scissors Stainless (Klappencker, Germany).

Methods and Results:

1. Cholinergic Agonism, Central/ Peripheral (Lippmann W and Pugsley TA. *Arch Int Pharmacodyn.* 227:324, 1977)

Test substance was administered orally to a group of 3 Wistar derived male or female rats weighing $150\pm20$ g. During the subsequent 30-60 minute period, the number of animals exhibiting more than 10 seconds of chewing

behavior (mouth and/or tongue movements) measured cumulatively and the number of animals exhibiting salivation or exhibiting salivation were noted. Positive responses observed in 2 or more (≥2) of 3 rats indicates possible central cholinergic activity and peripheral cholinergic activity.

Table 6: Result of Cholinergic Agonism, Central/peripheral in Rats

| Treatment | Route | Dose | N | Central | | Peripheral | |
|---|---|---|---|---|---|---|---|
| | | | | Chewing | Score | Salivation | Score |
| Vehicle | PO | 10 ml/kg | 1<br>2<br>3 | -<br>-<br>- | 0/3 | -<br>-<br>- | 0/3 |
| ACM-EtOH Extract | PO | 1000 mg/kg | 1<br>2<br>3 | +<br>+<br>+ | (3/3) | -<br>-<br>- | 0/3 |
| | | 300 mg/kg | 1<br>2<br>3 | -<br>-<br>+ | 1/3 | -<br>-<br>- | 0/3 |
| Arecoline-HBR | IP | 30 mg/kg | 1<br>2<br>3 | +<br>+<br>+ | (3/3) | +<br>+<br>+ | (3/3) |

Vehicle and test substances were administered orally (PO) while the positive reference compound was injected intraperitoneally (IP). During the subsequent 30-60 minute period, the number of animal exhibiting more than 10 seconds of chewing behavior (mouth and/or tongue movements) measured cumulatively or exhibiting salivation were noted. Positive responses observed in 2 or more (≥2) of 3 rats indicates possible cholinergic activity or peripheral cholinergic activity.

2. Cardiovascular, Blood Pressure and Heart rate (SHR 0, 1, 2, 4 hrs) (Yen TT et al. *Life Sci.* 22: 359, 1978)

Groups of 3 Wistar-Okamoto derived male spontaneously hypertensive rats (SHR) weighing 250±20 g were used; the mean systolic blood pressure was 200±20 mmHg and heart rate 400±30 beats/min. Blood pressure and heart rate were recorded indirectly by tail cuff method in a temperature controlled environment (32±1°C) before (0 time) and 1, 2 and 4 hours after oral administration of test substance or vehicle. A reduction in systolic pressure by 10 percent or more (≥10%), or decrease in heart rate by 20 percent or more (≥20%), at each measured time interval relative to 0 time, is considered significant. Table 7: Result of Cardiovascular, Blood Pressure (SHR 0, 1, 2, 4 Hours) in Rats

| Treatment | Route | Dose | N | % Control (from 0 times) | | |
|---|---|---|---|---|---|---|
| | | | | 1 Hour | 2 Hours | 4 Hours |
| Vehicle | PO | 10 ml/kg | 1<br>2<br>3 | 100<br>97<br>90 | 96<br>100<br>92 | 90<br>91<br>92 |
| | | | Ave. | 96 | 96 | 91 |
| ACM-EtOH Extract | PO | 1000 mg/kg | 1<br>2<br>3 | 78<br>86<br>89 | 85<br>89<br>89 | 71<br>80<br>89 |
| | | | Ave. | (84) | (88) | (80) |
| Clonidine | PO | 0.1 mg/kg | 1<br>2<br>3 | 71<br>95<br>72 | 67<br>86<br>85 | 71<br>88<br>69 |
| | | | Ave. | (79) | (79) | (76) |

Table 8: Result of Cardiovascular, Heart Rate (SHR 0, 1, 2, 4 Hours)

| Treatment | Route | Dose | N | % Control (from 0 times) | | |
|---|---|---|---|---|---|---|
| | | | | 1 Hour | 2 Hours | 4 Hours |
| Vehicle | PO | 10 ml/kg | 1<br>2<br>3 | 87<br>116<br>108 | 100<br>103<br>104 | 99<br>107<br>121 |
| | | | Ave. | 104 | 102 | 109 |
| ACM-EtOH Extract | PO | 1000 mg/kg | 1<br>2<br>3 | 98<br>81<br>83 | 93<br>100<br>78 | 95<br>88<br>92 |
| | | | Ave. | 87 | 90 | 92 |
| Clonidine | PO | 0.1 mg/kg | 1<br>2<br>3 | 62<br>84<br>68 | 97<br>87<br>86 | 112<br>104<br>78 |
| | | | Ave. | (71) | 90 | 98 |

SHR with systolic blood pressure of 200±20 mmHg and heart rates of 400±50 mmHg Beats/min were used. Blood pressure was recorded indirectly vial tail cuff at 0 time (before) and 1, 2 and 4 hours after oral administration of vehicle or test substance. A reduction in blood pressure by 10 percent or more (≥10%), or decrease in heart rate by 20 percent or more (≥20%) at each measurement time point relative to 0 time, shown in parenthesis, is considered significant.

Vehicle 10ml/kg 0 time 229mmHg and 403 mmHg beats/minute as 100%.

ACM-EtOH Extract 1000mg/kg 0 time 223mmHg and 452 mmHg beats/minute as 100%.

Clonidine 0.1mg/kg 0 time 228mmHg and 379 mmHg beats/minute as 100%

Table 9: Result of Cardiovascular, Blood Pressure (SHR 0, 1, 2, 4 Hours) in Rats

| Treatment | Route | Dose | N | % Control (from 0 times) | | |
|---|---|---|---|---|---|---|
| | | | | 1 Hour | 2 Hours | 4 Hours |
| Vehicle | PO | 10 ml/kg | 1<br>2<br>3 | 94<br>88<br>94 | 97<br>97<br>97 | 97<br>94<br>103 |
| | | | Ave. | 92 | 97 | 98 |
| ACM-EtOH Extract | PO | 300 mg/kg | 1<br>2<br>3 | 111<br>94<br>112 | 102<br>84<br>110 | 103<br>100<br>112 |
| | | | Ave. | 106 | 99 | 105 |
| Clonidine | PO | 0.1 mg/kg | 1<br>2<br>3 | 86<br>63<br>62 | 73<br>73<br>68 | 81<br>90<br>80 |
| | | | Ave. | (70) | (71) | (85) |

Table 10: Result of Cardiovascular, Heart Rate (SHR 0, 1, 2, 4 Hours)

| Treatment | Route | Dose | N | % Control (from 0 times) | | |
|---|---|---|---|---|---|---|
| | | | | 1 Hour | 2 Hours | 4 Hours |
| Vehicle | PO | 10 ml/kg | 1<br>2<br>3 | 82<br>88<br>109 | 85<br>115<br>111 | 84<br>102<br>119 |
| | | | Ave. | 93 | 104 | 102 |
| ACM-EtOH Extract | PO | 300 mg/kg | 1<br>2<br>3 | 97<br>105<br>85 | 96<br>108<br>96 | 92<br>98<br>82 |
| | | | Ave. | 96 | 100 | 91 |
| Clonidine | PO | 0.1 mg/kg | 1<br>2<br>3 | 77<br>78<br>62 | 85<br>78<br>94 | 102<br>100<br>104 |
| | | | Ave. | (72) | 86 | 102 |

SHR with systolic blood pressure of 200±20 mmHg and heart rates of 400±50 mmHg Beats/min were used. Blood pressure was recorded indirectly vial tail cuff at 0 time (before) and 1, 2 and 4 hours after oral administration of vehicle or test substance. A reduction in blood pressure by 10 percent or more (≥10%), or decrease in heart rate by 20 percent or more (≥20%) at each measurement time point relative to 0 time, shown in parenthesis, is considered significant.

Vehicle 10ml/kg 0 time 220mmHg and 410 mmHg beats/minute as 100%

ACM-EtOH Extract 300mg/kg 0 time 205mmHg and 446 mmHg beats/minute as 100%

Clonidine 0.1mg/kg 0 time 235mmHg and 417 mmHg beats/minute as 100%

3. Cholesterol, Serum (Total HDL, total/HDL, Ratio), Diet-Induced (Schurr PE et al., Atherosclerosis Drug Discovery. Plenum, New York, pp. 215-229, 1976)

Groups of 5 ICR derived male mice weighing 22±2 g were kept on a high fat diet (g/100g: coconut oil, 8; cholesterol, 1.0; cholic acid, 0.3; lard 2; standard chow 88.7) for 7 days to induce hypercholesterolemia. Test substance was administered orally on days 5, 6 and 7. After fasting overnight, serum was obtained from each mouse and assayed for total cholesterol (Total), high density lipoprotein (HDL) and percent change in Total/HDL. A decrease of 20 percent or more (≥20%) in serum Total or increase of 20 percent or more (≥20%) in serum HDL or decrease of 40% or more (≥40%) in the Total/HDL ratio relative to vehicle treated control animals is considered significant.

Table 11: Result of Cholesterol, (Total/HDL, Total/HDL Ratio), Diet-Induced in Mice

| Treatment | Route | Dose | N | Total | | HDL | | Total/HDL | |
|---|---|---|---|---|---|---|---|---|---|
| | | | | Indiv. | %Dce | Indiv. | %Dce | Indiv | %Dce |
| Vehicle | PO | 10 ml/kg × 3 | 1<br>2<br>3<br>4<br>5 | 361<br>316<br>379<br>392<br>367 | | 70<br>82<br>79<br>78<br>86 | | 5.16<br>3.85<br>4.80<br>5.03<br>4.27 | |
| | | | Ave. | 363 | -- | 79 | -- | 4.59 | -- |

(continued)

| Treatment | Route | Dose | N | Total | | HDL | | Total/HDL | |
|---|---|---|---|---|---|---|---|---|---|
| | | | | Indiv. | %Dce | Indiv. | %Dce | Indiv | %Dce |
| ACM-EtOH Extract | PO | 1000 mg/kg x 3 | 1<br>2<br>3<br>4<br>5 | 420<br>327<br>332<br>363<br>294 | | 117<br>115<br>104<br>98<br>117 | | 3.95<br>2.84<br>3.19<br>3.70<br>2.51 | |
| | | | Ave. | 347 | 4 | 110 | (39) | 3.15 | 31 |
| | PO | 300 mg/kg x 3 | 1<br>2<br>3<br>4<br>5 | 370<br>301<br>217<br>379<br>328 | | 66<br>65<br>74<br>76<br>98 | | 5.61<br>4.63<br>2.93<br>4.99<br>3.35 | |
| | | | Ave. | 319 | 12 | 76 | -4 | 4.20 | 8 |
| Benzafibrate PO | | 100 mg/kg x 3 | 1<br>2<br>3<br>4<br>5 | 230<br>214<br>225<br>231<br>242 | | 91<br>120<br>133<br>123<br>97 | | 2.53<br>1.78<br>1.69<br>1.88<br>2.49 | |
| | | | Ave. | 228 | (37) | 113 | (43) | 2.02 | (56) |

Vehicle, test substance or reference positive compound was administered orally (PO) on days 5, 6 and 7 after being fed a high cholesterol diet. Twenty-four hours after the third dose, the overnight-fasted test animals were sacrificed for assessing serum total cholesterol (Total) and high density lipoprotein (HDL). Decrease of 20 percent or more (≥20%) in serum Total or increase of 20 percent or more (≥20%) in serum HDL or decrease of 40% or more (≥40%) in the Total/HDL ratio is considered significant.

4. Hepatic Injury, D-Galactosamine (Wrobel J et al., *J. Med Chem* 41: 1084, 1998)

Groups of 5 Wistar derived male rats weighing 200±20 g were used. Each animal was treated with a single injection of D-galactosamine (500 mg/kg, IP) Test substance was administered orally at 0.5 hour before and 4 hours as well as 8 hours after D-galactosamine administration and animals were sacrificed 24 hours later. Serum alanine aminotransferase (ALT) and aspartate aminotransferase (AST) levels were measured by an optimized UV method with HITACHI automatic analyzer (model 7050). Reduction in ALT or AST activity by 30 percent or more (≥30%), relative to the vehicle treated control animal indicates significant protection.

Table 12: Result of Hepatic Injury, Galactosamine in Rats

| Treatment | Route | Dose | N | Serum ALT (X± SEM) | | Serum AST (X± SEM) | |
|---|---|---|---|---|---|---|---|
| | | | | U/L | Dec.% | U/L | Dec.% |
| Vehicle | PO | 10 ml/kg x 3 | 1<br>2<br>3<br>4<br>5 | 816<br>1044<br>652<br>656<br>644 | | 1628<br>1716<br>888<br>828<br>956 | |
| | | | X<br>SEM | 762.4<br>77.4 | -- | 1203.2<br>193.0 | -- |

(continued)

| Treatment | Route | Dose | N | Serum ALT (X± SEM) | | Serum AST (X± SEM) | |
|---|---|---|---|---|---|---|---|
| | | | | U/L | Dec.% | U/L | Dec.% |
| ACM-EtOH Extract | PO | 1000 mg/kg x 3 | 1<br>2<br>3<br>4<br>5 | 364<br>376<br>596<br>452<br>336 | | 516<br>532<br>672<br>524<br>356 | |
| | | | X<br>SEM | 424.8<br>46.9 | (44) | 520.0<br>50.1 | (57) |
| | | 300 mg/kg x 3 | 1<br>2<br>3<br>4<br>5 | 460<br>656<br>640<br>752<br>536 | | 852<br>880<br>876<br>1004<br>692 | |
| | | | X<br>SEM | 608.8<br>50.6 | 20 | 860.8<br>49.8 | 28 |
| Guanine | PO | 300 mg/kg x 3 | 1<br>2<br>3<br>4<br>5 | 508<br>532<br>412<br>436<br>636 | | 656<br>912<br>776<br>652<br>1028 | |
| | | | X<br>SEM | 504.8<br>39.6 | (34) | 804.8<br>73.4 | (33) |

Test substance and vehicle were administered orally at 0.5 hour before and 4, 8 hours after a single dose of galactosamine (500 mg/kg, IP). The rats were sacrificed 24 hours after galactosamine injection and the ALT and AST values were determined. A reduction of ≥30% in the ALT and AST relative to the vehicle group is considered significant.

5. Inflammation, Carrageenan (Winter CA et al., *Proc Soc Exp Biol Med.* 111:544, 1962)

A group of 3 Long Evans derived male or female overnight fasted rats weighing 150±20 g was fasted overnight prior to study. Test substance was administered orally one hour before right hind paw received injection of carrageenan (0.1 ml of 1 % suspension intraplantar). Hind paw edema, as a measure of inflammation, was recorded 3 hours after carrageenan administration using a plethysmometer with water cell (25 mm diameter). Reduction of hind paw edema by 30 percent or more (≥30%) indicates significant acute anti-inflammatory activity.

Table 13: Result of Inflammation, Carrageenan in Rats

| Treatment | Route | Dose | N | Paw Volume (x 0.01 ml) | | | % Inhibition |
|---|---|---|---|---|---|---|---|
| | | | | R.P. | L.P. | Diff | |
| Vehicle | PO | 10 ml/kg | 1<br>2<br>3 | 194<br>202<br>199 | 103<br>108<br>104 | 91<br>94<br>95 | |
| | | | Ave. | 198 | 105 | 93 | -- |
| ACM-EtOH Extract | PO | 1000 mg/kg | 1<br>2<br>3 | 146<br>147<br>160 | 101<br>95<br>104 | 45<br>52<br>56 | |
| | | | Ave. | 151 | 100 | 51 | (45) |

(continued)

| Treatment | Route | Dose | N | Paw Volume (x 0.01 ml) | | | % Inhibition |
|---|---|---|---|---|---|---|---|
| | | | | R.P. | L.P. | Diff | |
| Aspirin | PO | 150 mg/kg | 1<br>2<br>3 | 152<br>146<br>163 | 102<br>102<br>106 | 50<br>44<br>57 | |
| | | | Ave. | 154 | 103 | 50 | (46) |

Table 14: Result of Inflammation, Carrageenan in Rats

| Treatment | Route | Dose | N | Paw Volume (x 0.01 ml) | | | % Inhibition |
|---|---|---|---|---|---|---|---|
| | | | | R.P. | L.P. | Diff | |
| Vehicle | PO | 10 ml/kg | 1<br>2<br>3 | 193<br>198<br>199 | 105<br>107<br>102 | 88<br>91<br>97 | |
| | | | Ave. | 197 | 105 | 92 | -- |
| ACM-EtOH Extract | PO | 300 mg/kg | 1<br>2<br>3 | 195<br>187<br>196 | 104<br>103<br>103 | 91<br>84<br>93 | |
| | | | Ave. | 193 | 103 | 89 | 3 |
| Aspirin | PO | 150 mg/kg | 1<br>2<br>3 | 146<br>149<br>169 | 103<br>101<br>104 | 43<br>48<br>65 | |
| | | | Ave. | 155 | 103 | 52 | (43) |

Vehicle or test substance was administered to overnight fasted rats one hour before right hindpaw (R.P.) injection of carrageenan (0.1 ml of 1 % suspension intraplantar); the left hindpaw (L.P.) was not injected. Reduction of hindpaw edema by 30 percent or more (≥30%), shown in parenthesis, indicates significant acute anti-inflammatory activity.

6. Tumor, Syngeneic, Melanoma, B16-F0 Cell (Farrugia CA and Groves MJ. *Anticancer Research* 19: 1027-1032, 1999)

Groups of 5 immunocompetent (6-8 weeks old), pathogen-free (SPF) C57BL/6J male mice bred in an animal isolator (IVC racks) under specific pathogen free (SPF) condition were used. Viable B16-F0 murine melanoma cells (ATCC CRL-6322, 1.0 x $10^5$ in 0.2 ml), syngeneic for C57BL/6J mice, were injected subcutaneously into dorsal side of experimental mice. Treatment begins 24 hours after tumor inoculation and test compound was administered daily by oral gavage for 21 days, or less when overt signs of toxicity are seen. The mice were monitored for body weight, tumor size and survival starting from day 1 to day 22. Moreover, the tested mice were monitored for survival till the end of the study on day 45.

Tumor weight (mg) was estimated according to the formula for a prolate ellipsoid: length (mm) x [width (mm)]$^2$ x 0.5, assuming specific gravity to be one and π to be three. Tumor growth in compound treated animals was calculated as T/C (Treatment/Control) x 100%; a value of T/C ≤ 42% was considered significant in demonstrating antitumor activity.

The mean survival time of T/C (Treatment/Control) is ≥ 125% is also considered significant in demonstrating antitumor activity.

Table 15: Result of Tumor, Syngeneic, Melanoma B16-F0 Cell

| Treatment | Route | Dose | N | Tumor Weight (mg) and % T/C, Mean ± SEM | | | | | |
|---|---|---|---|---|---|---|---|---|---|
| | | | | Day 1. | T/C(%) | Day 8 | T/C(%) | Day 11 | T/C(%) |
| Vehicle | PO | 10 ml/kg x 21 | 1 | 0 | | 0 | | 60 | |
| | | | 2 | 0 | | 39 | | 298 | |
| | | | 3 | 0 | | 0 | | 49 | |
| | | | 4 | 0 | | 54 | | 541 | |
| | | | 5 | 0 | | 21 | | 117 | |
| | | | | 0 | 100 | 23±11 | 100 | 213± 93 | 100 |
| ACM-EtOH Extract | PO | 1000 mg/kg x 21 | 1 | 0 | | 0 | | 0 | |
| | | | 2 | 0 | | 0 | | 0 | |
| | | | 3 | 0 | | 0 | | 0 | |
| | | | 4 | 0 | | 0 | | 14 | |
| | | | 5 | 0 | | 0 | | 32 | |
| | | | | 0 | 100 | 0±0 | 0* | 9±6 | 4* |
| Mitomycin | IP | 2 mg/kg x 6 | 1 | 0 | | 0 | | 0 | |
| | | | 2 | 0 | | 0 | | 64 | |
| | | | 3 0 | | | 0 | | 0 | |
| | | | 4 | 0 | | 0 | | 68 | |
| | | | 5 | 0 | | 0 | | 41 | |
| | | | | 0 | 100 | 0±0 | 0* | 34±15 | 16* |

Table 16: Result of Tumor, Syngeneic, Melanoma B16-F0 Cell

| Treatment | Route | Dose | N | Tumor Weight (mg) and % T/C, Mean ± SEM | | | | | |
|---|---|---|---|---|---|---|---|---|---|
| | | | | Day 15 | T/C(%) | Day 18 | T/C(%) | Day 22 | T/C(%) |
| Vehicle | PO | 10 ml/kg x 21 | 1 | 211 | | 746 | | 2054 | |
| | | | 2 | 657 | | 1597 | | 2870 | |
| | | | 3 | 216 | | 669 | | 1419 | |
| | | | 4 | 835 | | 2455 | | 3688 | |
| | | | 5 | 240 | | 726 | | 1682 | |
| | | | | 432± 131 | 100 | 1239± 349 | 100 | 2343± 100 416 | |
| ACM-EtOH Extract | PO | 1000 mg/kg x 21 | 1 | 49 | | 280 | | 913 | |
| | | | 2 | 62 | | 630 | | 1545 | |
| | | | 3 | 388 | | 1079 | | 2560 | |
| | | | 4 | 148 | | 435 | | 1514 | |
| | | | 5 | 229 | | 535 | | 1637 | |
| | | | | 175± 62 | 41* | 592± 135 | 48 | 1634± 70 265 | |
| Mitomycin | IP | 2 mg/kg x 6 | 1 | 36 | | 256 | | 437 | |
| | | | 2 | 136 | | 849 | | 1248 | |
| | | | | 3 0 | | 0 | | 0 | |
| | | | 4 | 213 | | 525 | | 663 | |
| | | | 5 | 207 | | 327 | | Died | |
| | | | | 119± 44 | 27* | 391± 142 | 32* | 587± 260 | 25* |

Vehicle and test substance were administered to test animals every day at 24 hours after tumor cells implantation for a total of 21 doses. Concurrently, the reference compound, mitomycin, was administered IP twice a week for a total of 6 doses. Tumor size was measured and recorded twice a week for a period of 22 days. Tumor growth inhibition was calculated as T/C (treatment/control) x 100. A T/C value of ≤ 42% was considered significant in demonstrating antitumor activity.

Table 17: Result of Tumor, Syngeneic, Melanoma B16-F0 Cell

| Treatment | Route | Dose | N | Body Weight (g), Mean ± SEM | | | | | |
|---|---|---|---|---|---|---|---|---|---|
| | | | | Day 1 | Day 8 | Day 11 | Day 15 | Day 18 | Day 22 |
| Vehicle | PO | 10 ml/kg x 21 | 1<br>2<br>3<br>4<br>5 | 21<br>22<br>21<br>21<br>22 | 20<br>22<br>21<br>20<br>21 | 20<br>21<br>20<br>20<br>20 | 21<br>22<br>20<br>20<br>19 | 22<br>26<br>22<br>21<br>20 | 25<br>30<br>21<br>24<br>23 |
| | | | | 21.4± 0.2 | 20.8± 0.4 | 20.2± 0.2 | 20.4± 0.5 | 22.2± 1.0 | 24.6± 1.5 |
| ACM-EtOH Extract | PO | 1000 mg/kg x 21 | 1<br>2<br>3<br>4<br>5 | 20<br>20<br>20<br>21<br>20 | 21<br>19<br>18<br>20<br>20 | 21<br>19<br>18<br>19<br>21 | 22<br>21<br>19<br>21<br>22 | 22<br>22<br>21<br>20<br>23 | 23<br>24<br>26<br>21<br>25 |
| | | | | 20.2± 0.2 | 19.6± 0.5 | 19.6± 0.6 | 21.0± 0.5 | 21.6± 0.5 | 23.8± 0.9 |
| Mitomycin | IP | 2 mg/kg x 6 | 1<br>2<br>3<br>4<br>5 | 25<br>22<br>19<br>22<br>19 | 25<br>22<br>21<br>22<br>20 | 26<br>22<br>21<br>22<br>19 | 25<br>25<br>21<br>24<br>20 | 24<br>27<br>22<br>26<br>21 | 27<br>30<br>21<br>27<br>Died |
| | | | | 21.4± 1.1 | 22.2± 0.8 | 22.0± 1.1 | 23.0± 1.0 | 24.0± 1.1 | 26.3± 1.9 |

Vehicle and test substance were administered to test animals every day at 24 hours after tumor cells implantation for a total of 21 doses. Concurrently, the reference compound, mitomycin, was administered IP twice a week for a total of 6 doses. Tumor size was measured and recorded twice a week for a period of 22 days. The Student's t test was used to determine the siginifcant difference in the change of body weight between test compound and vehicle control group.

Table 18: Result of Tumor, Syngeneic, Melanoma B16-F0 Cell

| Treatment | Route | Dose | N | Survival Time (day), Mean ± SEM | |
|---|---|---|---|---|---|
| | | | | Days of Post-treatment | T/C (%) |
| Vehicle | PO | 10 ml/kg x 21 | 1<br>2<br>3<br>4<br>5 | 23<br>28<br>30<br>28<br>27 | |
| | | | | 27.2 ±1.2 | 100 |

(continued)

| Treatment | Route | Dose | N | Survival Time (day), Mean ± SEM | |
|---|---|---|---|---|---|
| | | | | Days of Post-treatment | T/C (%) |
| ACM-EtOH Extract | PO | 1000 mg/kg x 21 | 1<br>2<br>3<br>4<br>5 | 44<br>31<br>25<br>42<br>32 | |
| | | | | 34.8±3.6 | 128* |
| Mitomycin | IP | 2 mg/kg x 6 | 1<br>2<br>3<br>4<br>5 | 45[a]<br>28<br>45[a]<br>30<br>22 | |
| | | | | 34.0±4.7 | 125* |
| a: The animal did not die through day 45 and the survival day was served as 45 days. | | | | | |

The treated mice were monitored for survival through the end of the study on day 45 or the day when test animal died. The mean survival time of T/C (Treatment/Control) ≥125% is also considered significant in demonstrating anti-tumor activity.

Discussion:

ACM-EtOH Extract, administered orally (PO), in accordance with in-house established criteria, caused significant activities in the following mouse and rat assays:

Central cholinergic agonism at 1000 mg/kg in rats; a minimum and non-significant agonism was seen a 300 mg/kg; no significant agonism or antagonism on peripheral cholinergic nerve was seen at 1000 mg/kg (Table 6)

Decrease in systolic blood pressure (16%, 12 % and 20% at respective 1, 2 and 4 hours observation time points vs. 100 % with 0 time) and associated moderate but non-significant decrease in heart rate at 1000 mg/kg in spontaneously hypertensive (SH) rats (Tables 7 and 8); dose of 300 mg/kg did not cause significant changes in systolic blood pressure nor the heart rate (Tables 9 and 10)

Increase in high density lipoprotein (HDL, 39% over vehicle control) at 1000 mg/kg in diet-induced mice (Table 10); the associate total cholesterol (Total) did not change significantly, while the HDL/total ratio was decreased to near significant 31 %; dose of 300 mg/kg did not cause significant changes in Total, HDL and HDL/Total ratio (Table 11).

Hepatoprotection (44% decrease in ALT and 57% decrease in AST vs. vehicle control) from galactosamine induced hepatic injury in rats at 1000 mg/kg x 3; moderate decrease of 20 %in ALT and of 28 % in AST at 300 mg/kg x 3 was seen (Table 12)

Anti-inflammation (45% inhibition vs. vehicle control) versus carrageenan-induced paw edema in rats at 1000 mg/kg (Table 13); the lower level of 300 mg/kg did not demonstrate significant activity (3% inhibition vs. vehicle, Table 14)

Anti-tumor activity in syngeneic melanoma B16-F0 cell for C57BL/6J mice on day 8, 11 and 15 (Tables 15 and 16) as well as prolongation in animal survival time at 1000 mg/kg (Table 18); animal body weight did not change significantly (Table 17).

**Tests for ACM, ACM-EtOH Extract and Compound 3 of the Invention**

Nine groups of ICR derived male mice (weighing 22±2 g) of 5 each were used. Each animal was challenged with a single dose of carbon tetrachloride ($CCl_4$, 0.1 ml/kg in 50% olive oil, PO). The test substance of ACM at doses 300 and 1000 mg/kg or compound 3 of the invention at doses 30, 100 and 300 mg/kg were administered orally at 30 minutes before and 4, 8 hours after $CCl_4$ challenge; whereas ACM-ETOH Extract at 300 and 1000 mg/kg administered orally was pretreated one day (twice a day) and 30 minutes before and 4, 8 hours after carbon tetrachloride The animals were sacrificed 24 hours after $CCl_4$. Alanine aminotransferase (ALT) and Aspartate aminotransferase (AST) levels were

measured by optimized UV method using a HITACHI automatic analyzer (model 7050). A reduction of ALT or AST levels by 30 percent or greater (≥30%), relative to the vehicle group, indicating significant protection from hepatic injury.

Results

Table 19: Assay Hepatic Injury, Carbon Tetrachloride, in Mice

| Treatment | Route | Dose | N | ALT | | AST | |
|---|---|---|---|---|---|---|---|
| | | | | U/L Dec.% | | U/L Dec.% | |
| Vehicle (2% Tween 80) | PO | 10 ml/kg x 3 | 1 | 3936 | | 2056 | |
| | | | 2 | 3456 | | 1856 | |
| | | | 3 | 3712 | | 1528 | |
| | | | 4 | 2560 | | 1328 | |
| | | | 5 | 2968 | | 1696 | |
| | | | X | 3326 | 0 | 1693 | 0 |
| | | | SEM | 250 | | 126 | |
| ACM | PO | 1000 mg/kg x 3 | 1 | 1440 | | 888 | |
| | | | 2 | 2720 | | 1520 | |
| | | | 3 | 2272 | | 1328 | |
| | | | 4 | 1272 | | 792 | |
| | | | 5 | 1320 | | 880 | |
| | | | X | 180.5 | (46) | 1082 | (36) |
| | | | SEM | 292 | | 144 | |
| | | 300 mg/kg x 3 | 1 | 2336 | | 1256 | |
| | | | 2 | 1552 | | 1072 | |
| | | | 3 | 3720 | | 1512 | |
| | | | 4 | 3816 | | 2336 | |
| | | | 5 | 3952 | | 2792 | |
| | | | X | 3075 | 8 | 1794 | -6 |
| | | | SEM 480 | | | 330 | |
| ACM-EtOH Extract | PO | 1000 mg/kg x 5 | 1 | 1936 | | 1232 | |
| | | | 2 | 1528 | | 768 | |
| | | | 3 | 1896 | | 1136 | |
| | | | 4 | 2752 | | 1656 | |
| | | | 5 | 2472 | | 1592 | |
| | | | X | 2117 | (36) | 1277 | 25 |
| | | | SEM | 219 | | 162 | |
| | | 300 mg/kg x 5 | 1 | 1656 | | 976 | |
| | | | 2 | 3536 | | 1712 | |
| | | | 3 | 2328 | | 1808 | |
| | | | 4 | 1736 | | 1416 | |
| | | | 5 | 1792 | | 888 | |
| | | | X | 2210 | (34) | 1360 | 20 |
| | | | SEM | 352 | | 187 | |

(continued)

| Treatment | Route | Dose | N | ALT | | AST | |
|---|---|---|---|---|---|---|---|
| | | | | U/L Dec.% | | U/L Dec.% | |
| Compound 3 | PO | 300 mg/kg x 3 | 1<br>2<br>3<br>4<br>5 | 1368<br>1576<br>1440<br>2728<br>2720 | | 776<br>896<br>896<br>1352<br>1728 | |
| | | | X<br>SEM | 1966<br>311 | (41) | 1130<br>179 | (33) |
| | | 100 mg/kg x 3 | 1<br>2<br>3<br>4<br>5 | 3200<br>4576<br>2512<br>2728<br>3696 | | 2256<br>2976<br>1536<br>1552<br>1600 | |
| | | | X<br>SEM | 3342<br>370 | 0 | 1984<br>282 | -17 |
| | | 30 mg/kg x 3 | 1<br>2<br>3<br>4<br>5 | 4296<br>3696<br>2152<br>2400<br>4256 | | 2136<br>2288<br>1096<br>1792<br>2496 | |
| | | | X<br>SEM | 3360<br>457 | -1 | 1962<br>245 | -16 |
| Silymarin | PO | 100 mg/kg x 3 | 1<br>2<br>3<br>4<br>5 | 2856<br>1832<br>1296<br>2792<br>2728 | | 1296<br>1152<br>952<br>1072<br>1336 | |
| | | | X<br>SEM | 2301<br>313 | (31) | 1162<br>71 | (31) |

Discussion:

ACM, ACM-EtOH Extract and compound **3** of the invention were evaluated for possible protective activity from hepatic injury induced by carbon tetrachloride in ICR mice. The test substance of ACM at doses 300 and 1000 mg/kg and compound **3** of the invention at doses 30, 100 and 300 mg/kg were administered orally to test animals 0.5 hour before and 4, 8 hours after $CCl_4$ challenge. For ACM-EtOH Extract at 300 and 1000 mg/kg, 2 times (b.i.d.) of treatment (9:00 AM and 16:00 PM) were done 1 day before $CCl_4$ and followed 0.5 hr before and 4, 8 hours after $CCl_4$ challenge (5 dosing in total). The degree of hepatic injury was determined by increase in serum alanine aminotransferase (ALT) and aspartate aminotransferase (AST) levels relative to the vehicle treated animals. ACM at 1000 mg/kg x 3 and compound 3 of the invention at 300 mg/kg x 3 caused a significant reduction of ALT (46% and 41%) and AST (36% and 33%) relative to the vehicle treated animals. Simultaneously, ACM-EtOH Extract at 300 and 1000mg/kg x 5 also caused significant reduction in ALT (36% and 34%) and AST (25% and 20%).

Concurrently tested silymarin (100 mg/kg x 3, IP) showed significantly reduction of ALT (31 %) and AST (31 %) relative to the vehicle treated group.

It is concluded that ACM, ACM-EtOH Extract and compound **3** of the invention possess the ability of significant hepatoprotectant activity in a mouse $CCl_4$ model.

**Protective effect of *Antrodia camphorata* and compound 3 on fulminant hepatitis**

One of the animal models of human viral hepatitis is *Propionibacterium* acnes-lipopolysaccharide (*P. acnes-LPS*) induced mouse hepatitis. This mouse experimental hepatitis model is widely accepted for studying fulminant hepatitis such as human viral hepatitis. We have taken this animal model to evaluate the protective efficacy of extracts of *Antrodia camphorata* and compound 3 on fulminant hepatitis. We assessed the effectiveness of the chemicals and the extracts by measuring the serum concentration of glutamic oxaloactic transminase (GOT), glutamic pyruvic transaminase (GPT), total protein, and albumin in the hepatitis mice.

Methods and Equipments

Animals

ICR mice were purchased from SLC Co., Ltd. (Shizuoka, Japan). They were kept in an air-conditioned animal room and took water and feed *ad libitum.* Animal quarantine period was longer than one week.

*P. acnes,* ATCC 6919, was purchased from Science Research Institute in Saitama Japan. It was cultured in a medium which contains brain heart infusion, L-cystein (0.03%), Tween80 (0.03%) in distilled water under anaerobic condition at 37°C for 48 hours. At the termination of the culture, *P. acnes* were spun down at 7000 rpm and 4°C for 15 minutes. After spinning, the collected *P. acnes* were re-suspended with PBS and was spun down again. Then the collected P. acnes was suspended again with PBS. The suspension solution was heated at 80°C for 30 minutes and freeze-dried to prepare powder.

Fractionation of mycelium of *Antrodia camphorata*

Thirty gram of *Antrodia camphorata* mycelia (Lot #: C071202-1) was mixed with 100ml chloroform. The mixture was extracted by refluxing at 40°C for 1 hour. The reflux procedure was repeated three times. All extracts were combined and prepared as freeze-dried powder. The final volume of 4.5g of powder was obtained. The residue of chloroform extraction was refluxed with 100ml boiling water for 1 hour. The procedure was also repeated three times. All water extracts were freeze-dried.

Compound 3 was isolated and purified as described before.

Administration

The water extract was dissolved in distilled water. The chloroform extract and compound 3 were suspended in distilled water with 4% Tween 80.

Serum measurement

Sera were separated by centrifugation blood at 3000rpm, 4°C for 15 minutes. GOT and GPT were measured by using transaminase CII-Test Wako (Wako Jyun-Yaku Co., Ltd. Osaka). Total protein and albumin were measured by using A/G B-Test Wako (Wako Jyun-Yaku Co., Ltd. Osaka).

Experiment Procedures

The mouse fulminant hepatitis was induced as follows: ICR male mice (8 weeks old) received 0.5mg of heat-killed *Propionibacterium acnes* (*P. acnes*) by intravenous injection. On the 8th days after the *P. acnes* injection, mice were challenged with 0.25mg of LPS by intravenous injection to induce fulminate hepatitis. Extracts of *Antrodia camphorata* and compound 3 were given to mice orally by a gastric tube once a day for eight consecutive days right after *P. acnes* injection. Thereafter, in order to assess the effect of the test substances, mice sera were collected at 18 hours after the LPS challenge.

Results

The survival rate of the control group (mice administered with water) was 30%. Mice that received water extract of *Antrodia camphoarata* at doses of 200mg/kg and 50mg/kg showed survival rates of 60% and 40%, respectively.

The average GOT titer of the control mice was 1662 IU/L, and the average GOT titers of mice treated with 200mg/kg, and 50mg/kg water extract were 208IU/L and 1159IU/L, respectively. The average GPT titers of the control mice, mice treated with 200mg/kg and 50mg/kg water extract of *Antrodia camphoarata* were 1256IU/L, 193IU/L, and

697IU/L, respectively.

The concentration of total protein and albumin in fulminate hepatitis mice was also reduced in mice treated with water extract of *Antrodia camphoarata* compared to the control mice. The concentration of total protein of mice treated with 200mg/kg water extract of *Antrodia camphoarata* the total protein concentration recovered to the normal level, but not the concentration of albumin.

Compound 3 also showed potent protective efficacy on fulminant hepatitis. The survival rate was 67% in hepatitis mice treated with compound 3 20mg/kg/day for 8 days. The GOT and GPT levels of the same group were 299IU/L and 153IU/L, respectively. The percentage of GOT and GPT inhibited of mice treated with compound 3 were 82% and 88%, respectively. Other indexes of mice treated with compound 3, such as the concentrations of the total protein and albumin, were similar to the control.

These experimental results (Figure 8) suggested that water extract of mycelium of *Antrodia camphorata* and compound 3, an active chemical from *Antradia camphoarata,* have a potent hepato-protective effect against fulminant hepatitis.

Other embodiments are set forth within the following claims.

## Claims

1. A compound having the formula

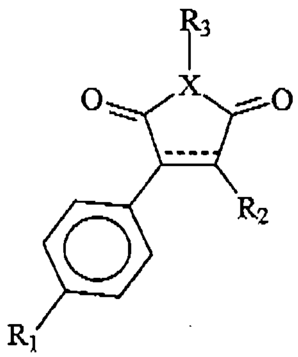

its tautomeric forms, its stereoisomers, its pharmaceutically acceptable salts, or its pharmaceutically acceptable solvates,
wherein
X is N or O;
$R_1$ is $C_{1-10}$ alkyloxy, $C_{2-10}$ alkenyloxy, or $C_{2-0}$ alkynyloxy;
$R_2$ is H, $C_{1-10}$ alkyl, $C_{2-10}$ alkenyl or $C_{1-10}$ alkynyl;
$R_3$ is absent, H, hydroxyl or $C_{1-10}$ alkyl;
----- denotes a single or double bond;
provided that
if X is O, $R_3$ is absent;
if ----- denotes a single bond, the compound has the formula:

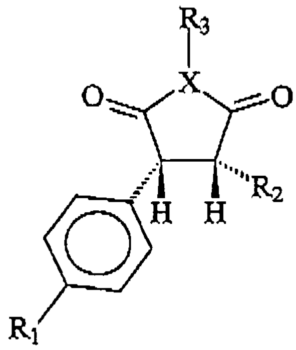

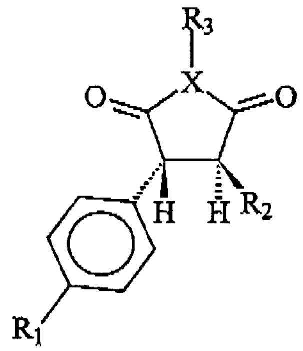

,

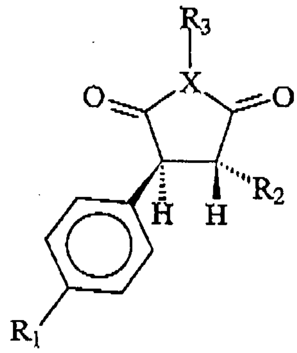

or

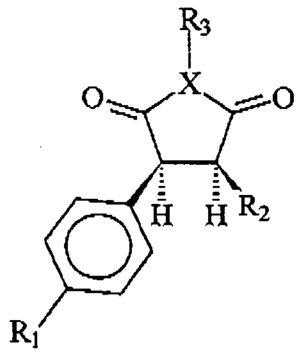

,

for use in a method of:

(a) decreasing blood pressure;
(b) increasing high density lipoprotein;
(c) central cholinergic agonism;
(d) providing hepatoprotection against D-galactosamine or Hepatitis C virus; or
(e) treating inflammation.

**2.** The compound of Claim 1, wherein $R_1$ is $C_{2-6}$ alkenyloxy or $C_{2-6}$ alkynyloxy.

**3.** The compound of Claim 2, wherein $C_{2-6}$ alkenyloxy is substituted with $C_{1-6}$ alkyl.

**4.** The compound of Claim 1, wherein $R_2$ is isobutyl.

**5.** The compound of Claim 1, which is 3-isobutyl-4-[4-(3-methyl-2-butenyloxy)phenyl]furan-2,5-dione,3-isobutyl-4-[4-(3-methyl-2-butenyloxy)phenyl]-1*H*-pyrrol-2,5-dione,3-isobutyl-4-[4-(3-methyl-2-butenyloxy)phenyl]-1*H*-pyrrol-1*H*-ol-2,5-dione, *3R*,4S**-1-hydroxy-3-isobutyl-4-[4-(3-methyl-2-butenyloxy)phenyl]pyrrolidine-2,5-dione, or *3R*,4R**-1-hydroxy-3-isobutyl-4-[4-(3-methyl-2-butenyloxy)phenyl]pyrrolidine-2,5-dione.

**6.** A pharmaceutical composition comprising:

(i) a compound having the formula

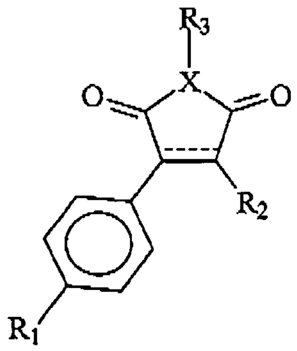

its tautomeric forms, its stereoisomers, its pharmaceutically acceptable salts, or its pharmaceutically acceptable solvates,
wherein
X is N or O;
$R_1$ is $C_{1-10}$ alkyloxy, $C_{2-10}$ alkenyloxy, or $C_{2-10}$ alkynyloxy;
$R_2$ is H, $C_{1-10}$ alkyl, $C_{2-10}$ alkenyl or $C_{2-10}$ alkynyl;
$R_3$ is absent, H, hydroxyl or $C_{1-10}$ alkyl;
----- denotes a single or double bond;
provided that
if X is O, $R_3$ is absent;
if ----- denotes a single bond, the compound has the formula:

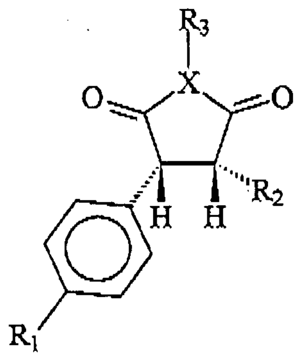

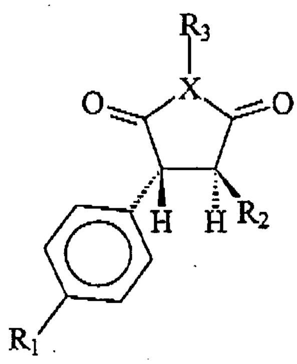

,

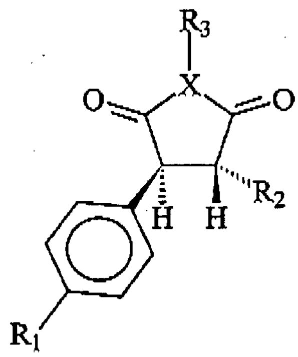

or

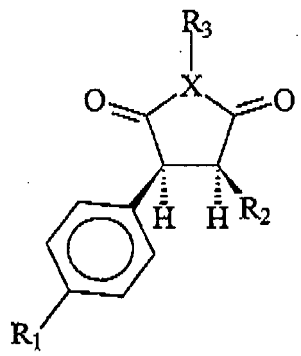

;and
(ii) a pharmaceutically acceptable carrier or diluent, for use in a method of:

(a) decreasing blood pressure;
(b) increasing high density lipoprotein;
(c) central cholinergic agonism;

(d) providing hepatoprotection against D-galactosamine or Hepatitis C virus; or
(e) treating inflammation.

**7.** The composition of Claim 6, wherein the compound is 3-isobutyl-4-[4-(3-methyl-2-butenyloxy)phenyl]furan-2,5-dione, 3-isobutyl-4-[4-(3-methyl-2-butenyloxy)phenyl]-1*H*-pyrrol-2,5-dione, 3-isobutyl-4-[4-(3-methyl-2-butenyloxy)phenyl]-1*H*-pyrrol-1-ol-2,5-dion, *3R**,*4S**-1-hydroxy-3-isobutyl-4-(4-(3-methyl-2-butenyloxy)phenyl]pyrrolidine-2,5-dione, or *3R**,*4R**-1-hydroxy-3-isobutyl-4-[4-(3-methyl-2-butenyloxy)phenyl]pyrrolidine-2,5-dione.

**8.** A mixture from mycelium of *Antrodia camphorate* for:

(a) decreasing blood pressure;
(b) increasing high density lipoprotein;
(c) central cholinergic agonism;
(d) hepatoprotection against D-galactosamine or Hepatitis C virus; or
(e) treating inflammation,

wherein the mycelium has been previously prepared according to submerged liquid fermentation and wherein the mixture is prepared from water or organic solvent extract of said mycelium.

**9.** The mixture of Claim 8, wherein the organic solvent is alcohol, ester, alkane or halogenated alkane.

**10.** The mixture of Claim 9, wherein the alcohol is ethanol.

**11.** A mycelium of *Antrodia camphorata* for:

(a) decreasing blood pressure;
(b) increasing high density lipoprotein;
(c) central cholinergic agonism;
(d) hepatoprotection against D-galactosamine; Hepatitis C virus; or
(e) treating inflammation,

wherein the mycelium has been previously prepared according to submerged liquid fermentation.

**12.** The compound of claim 1 or the composition of claim 6, wherein central cholinergic agonism is used for treating or preventing Alzheimer's disease.

**13.** Use of a compound as defined in claim 1, or a pharmaceutical composition as defined in claim 6, or a mixture as defined in claim 8, or a mycelium as defined in claim 11, for the manufacture of a medicament for:

(a) decreasing blood pressure;
(b) increasing high density lipoprotein;
(c) central cholinergic agonism;
(d) hepatoprotection against D-galactosamine or Hepatitis C virus; or
(e) treating inflammation.

**Patentansprüche**

**1.** Verbindung, welche die Formel

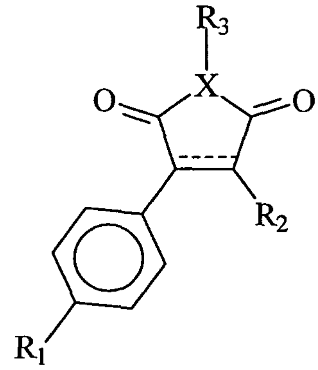

hat, oder ihre tautomeren Formen, ihre Stereoisomere, ihre pharmazeutisch verträglichen Salze, oder ihre pharmazeutisch verträglichen Solvate,
wobei
X N oder O ist;
$R_1$ $C_{1-10}$ Alkyloxy, $C_{2-10}$ Alkenyloxy, oder $C_{2-10}$ Alkinyloxy ist;
$R_2$ H, $C_{1-10}$ Alkyl, $C_{2-10}$ Alkenyl oder $C_{2-10}$ Alkinyl ist;
$R_3$ fehlt oder H, Hydroxyl oder $C_{1-10}$ Alkyl ist;
----- eine Einfach- oder Doppelbindung darstellt;
mit dem Vorbehalt, dass,
falls X O ist, $R_3$ fehlt;
falls ----- eine Einfachbindung darstellt, die Verbindung die Formel

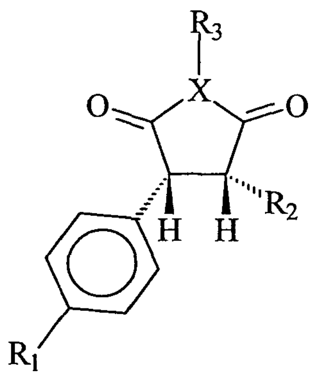

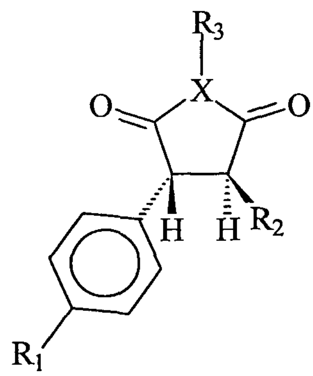

,

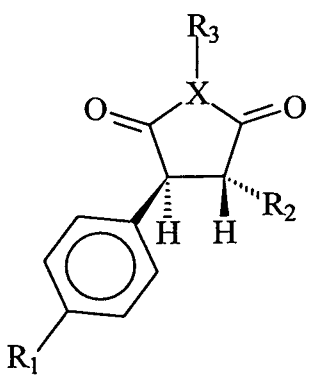

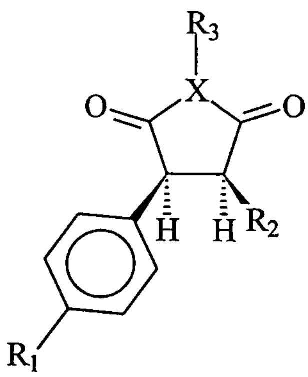

oder

hat,
zur Verwendung in einem Verfahren

(a) zum Blutdrucksenken;
(b) zum Erhöhen des Lipoproteins hoher Dichte (HDL);
(c) zum zentralcholinergen Agonismus;
(d) zur Bereitstellung von Schutz der Leber vor D-Galactosamin oder Hepatitis C Virus; oder
(e) zur Behandlung von Entzündung.

**2.** Verbindung nach Anspruch 1, wobei $R_1$ $C_{2-6}$ Alkenyloxy or $C_{2-6}$ Alkinyloxy ist.

**3.** Verbindung nach Anspruch 2, wobei $C_{2-6}$ Alkenyloxy durch $C_{1-6}$Alkyl substituiert ist.

**4.** Verbindung nach Anspruch 1, wobei $R_2$ Isobutyl ist.

**5.** Verbindung nach Anspruch 1, welche 3-Isobutyl-4-[4-(3-methyl-2 butenyloxy)phenyl]furan-2,5-dion, 3-Isobutyl-4-[4-(3-methyl-2-butenyloxy)phenyl]-1*H*-pyrrol-2,5-dion, 3-Isobutyl-4-[4-(3-methyl-2-butenyloxy)phenyl]-1*H*-pyrrol-1-ol-2,5-dion, *3R**, *4S**-1-Hydroxy-3-isobutyl-4-[4-(3-methyl-2-butenyloxy)phenyl]pyrrolidin-2,5-dion, oder *3R**, *4R**-1-Hydroxy-3-isobutyl-4-[4-(3-methyl-2-butenyloxy)phenyl]pyrrolidin-2,5-dion ist.

**6.** Pharmazeutische Zusammensetzung, welche:

(i) eine Verbindung aufweist, welche die Formel

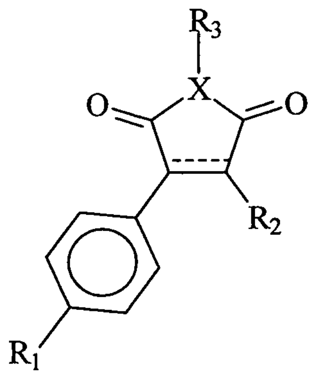

hat, oder ihre tautomeren Formen, ihre Stereoisomere, ihre pharmazeutisch verträglichen Salze, oder ihre pharmazeutisch verträglichen Solvate,
wobei
X N oder O ist;
$R_1$ $C_{1-10}$ Alkyloxy, $C_{2-10}$ Alkenyloxy, oder $C_{2-10}$ Alkinyloxy ist;
$R_2$ H, $C_{1-10}$ Alkyl, $C_{2-10}$ Alkenyl oder $C_{2-10}$ Alkinyl ist;
$R_3$ fehlt oder H, Hydroxyl oder $C_{1-10}$ Alkyl ist;
----- eine Einfach- oder Doppelbindung darstellt;
mit dem Vorbehalt, dass
falls X O ist, $R_3$ fehlt;
falls ----- eine Einfachbindung darstellt, die Verbindung die Formel

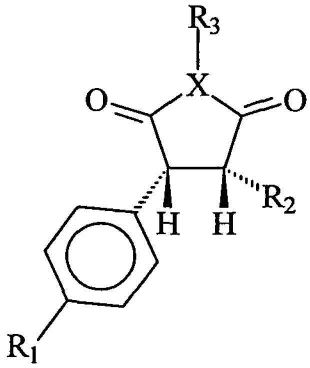

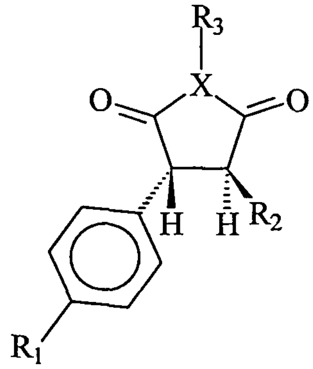

,

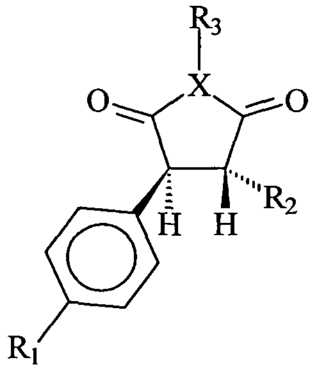

oder

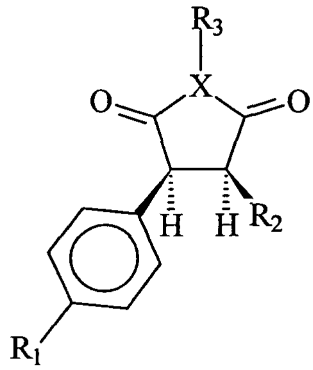

hat; und
(ii) einen pharmazeutisch verträglichen Träger oder Verdünnungsmittel aufweist, zur Verwendung in einem Verfahren

(a) zum Blutdrucksenken;
(b) zum Erhöhen des Lipoproteins hoher Dichte (HDL);
(c) zum zentralcholinergen Agonismus;
(d) zur Bereitstellung von Schutz der Leber vor D-Galactosamin oder Hepatitis C Virus; oder
(e) zur Behandlung von Entzündung.

**7.** Zusammensetzung nach Anspruch 6, wobei die Verbindung 3-Isobutyl-4-[4-(3-methyl-2-butenyloxy)phenyl]furan-2,5-dion, 3-Isobutyl-4-[4-(3-methyl-2-butenyloxy)phenyl]-1*H*-pyrrol-2,5-dion, 3-Isobutyl-4-[4-(3-methyl-2-butenyloxy)phenyl]-1*H*-pyrrol-1-ol-2,5-dion, *3R**,*4S**-1-Hydroxy-3-isobutyl-4-[4-(3-methyl-2-butenyloxy)phenyl]pyrrolidin-2,5-dion, oder *3R**,*4R**-1-Hydroxy-3-isobutyl-4-[4-(3-methyl-2-butenyloxy)phenyl]pyrrolidin-2,5-dion ist.

**8.** Mischung aus Myzel aus *Antrodia camphorata*

(a) zum Blutdrucksenken;
(b) zum Erhöhen des Lipoproteins hoher Dichte (HDL);
(c) zum zentralcholinergen Agonismus;
(d) zum Schutz der Leber vor D-Galactosamin oder Hepatitis C Virus; oder
(e) zur Behandlung von Entzündung,

wobei das Myzel zuvor gemäß Submersflüssigfermentation hergestellt wurde und wobei die Mischung aus Wasser oder organischem Lösungsmittelextrakt aus dem Myzel hergestellt wurde.

**9.** Mischung nach Anspruch 8, wobei das organische Lösungsmittel Alkohol, Ester, Alkan oder halogeniertes Alkan ist.

**10.** Mischung nach Anspruch 9, wobei der Alkohol Ethanol ist.

**11.** Myzel aus *Antrodia camphorata*

(a) zum Blutdrucksenken;
(b) zum Erhöhen des Lipoproteins hoher Dichte (HDL);
(c) zum zentralcholinergen Agonismus;
(d) zum Schutz der Leber vor D-Galactosamin oder Hepatitis C Virus; oder
(e) zur Behandlung von Entzündung,

wobei das Myzel zuvor gemäß Submersflüssigfermentation hergestellt wurde.

**12.** Verbindung nach Anspruch 1 oder Zusammensetzung nach Anspruch 6, wobei zentralcholinerger Agonismus verwendet wird, um Alzheimer'sche Krankheit zu behandeln oder vorzubeugen.

**13.** Verwendung einer Verbindung wie in Anspruch 1 definiert, oder einer pharmazeutischen Zusammensetzung wie in Anspruch 6 definiert, oder einer Mischung wie in Anspruch 8 definiert oder eines Myzels wie in Anspruch 11 definiert zur Herstellung eines Medikaments

(a) zum Blutdrucksenken;
(b) zum Erhöhen des Lipoproteins hoher Dichte (HDL);
(c) zum zentralcholinergen Agonismus;
(d) zum Schutz der Leber vor D-Galactosamin oder Hepatitis C Virus; oder
(e) zur Behandlung von Entzündung.

## Revendications

**1.** Composé de formule :

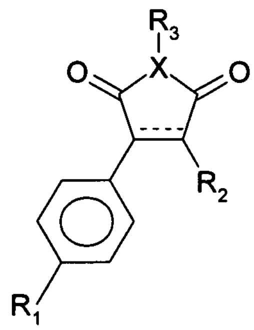

ainsi que ses formes tautomères, ses stéréoisomères, ses sels pharmacologiquement admissibles et ses solvats pharmacologiquement admissibles,
dans laquelle formule :

X représente un atome d'azote ou d'oxygène ;
$R_1$ représente un groupe alcoxy en $C_{1-10}$, alcényl-oxy en $C_{2-10}$ ou
alcynyl-oxy en $C_{2-10}$ ;
$R_2$ représente un atome d'hydrogène ou un groupe alkyle en $C_{1-10}$, alcényle en $C_{2-10}$ ou alcynyle en $C_{2-10}$ ;
$R_3$ ne représente rien ou représente un atome d'hydrogène, un groupe hydroxyle ou un groupe alkyle en $C_{1-10}$ ;
et ---- représente une simple liaison ou une double liaison ;

étant entendu que :

si X représente un atome d'oxygène, $R_3$ ne représente rien ;
et si ---- représente une simple liaison, le composé présente l'une des formules suivantes :

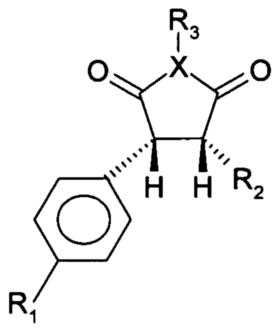 , 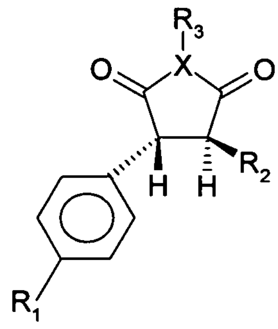 ,

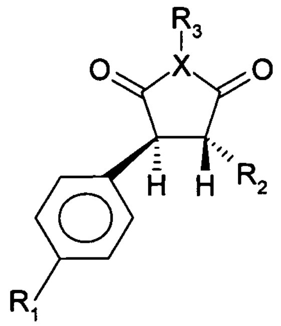 ou 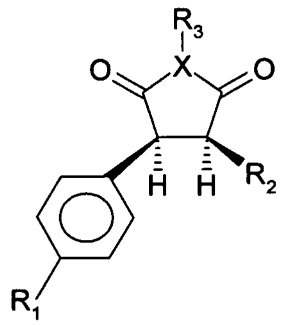 ;

pour utilisation dans un procédé visant :

a) à faire baisser la pression sanguine ;
b) à faire augmenter le taux de lipoprotéines de haute densité ;
c) à induire un agonisme cholinergique central ;
d) à fournir au foie une protection contre la D-galactosamine ou le virus de l'hépatite C ;
e) ou à traiter une inflammation.

**2.** Composé conforme à la revendication 1, dans lequel $R_1$ représente un groupe alcényl-oxy en $C_{2-6}$ ou alcynyl-oxy en $C_{2-6}$.

**3.** Composé conforme à la revendication 2, dans lequel le groupe alcényl-oxy en $C_{2-6}$ porte un substituant alkyle en $C_{1-6}$.

**4.** Composé conforme à la revendication 1, dans lequel $R_2$ représente un groupe isobutyle.

**5.** Composé conforme à la revendication 1, qui est l'un des suivants :

3-isobutyl-4-[4-(3-méthyl-but-2-ényl-oxy)-phényl]-furane-2,5-dione,
3-isobutyl-4-[4-(3-méthyl-but-2-ényl-oxy)-phényl]-1-H-pyrrole-2,5-dione,
1-hydroxy-3-isobutyl-4-[4-(3-méthyl-but-2-ényl-oxy)-phényl]-1H-pyrrole-2,5-dione,
*3R**,*4S**-1-hydroxy-3-isobutyl-4-[4-(3-méthyl-but-2-ényl-oxy)-phényl]-pyrrolidine-2,5-dione,
*3R**,*4R**-1-hydroxy-3-isobutyl-4-[4-(3-méthyl-but-2-ényl-oxy)-phényl]-pyrrolidine-2,5-dione.

**6.** Composition pharmaceutique comprenant :

i) un composé de formule :

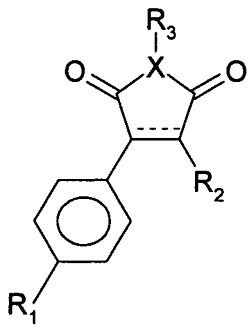

ou l'un ou l'une de ses formes tautomères, ses stéréoisomères, ses sels pharmacologiquement admissibles et ses solvats pharmacologiquement admissibles,
dans laquelle formule :

X représente un atome d'azote ou d'oxygène ;
$R_1$ représente un groupe alcoxy en $C_{1-10}$, alcényl-oxy en $C_{2-10}$ ou alcynyl-oxy en $C_{2-10}$ ;
$R_2$ représente un atome d'hydrogène ou un groupe alkyle en $C_{1-10}$, alcényle en $C_{2-10}$ ou alcynyle en $C_{2-10}$ ;
$R_3$ ne représente rien ou représente un atome d'hydrogène, un groupe hydroxyle ou un groupe alkyle en $C_{1-10}$ ;
et ---- représente une simple liaison ou une double liaison ;

étant entendu que :

si X représente un atome d'oxygène, $R_3$ ne représente rien ;
et si ---- représente une simple liaison, le composé présente l'une des formules suivantes :

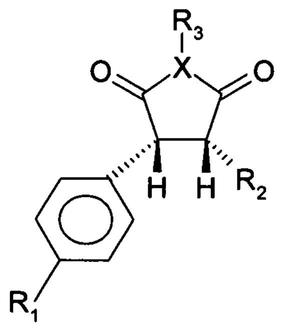 , 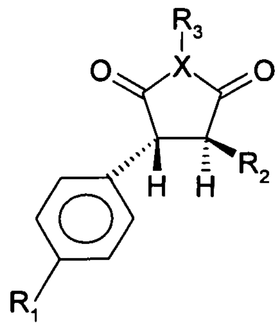 ,

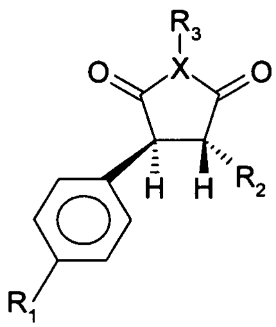 ou 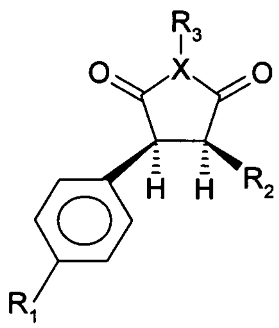 ;

ii) et un véhicule ou diluant pharmacologiquement admissible,
pour utilisation dans un procédé visant :

a) à faire baisser la pression sanguine ;
b) à faire augmenter le taux de lipoprotéines de haute densité ;
c) à induire un agonisme cholinergique central ;
d) à fournir au foie une protection contre la D-galactosamine ou le virus de l'hépatite C ;
e) ou à traiter une inflammation.

**7.** Composition conforme à la revendication 6, dans laquelle le composé est l'un des suivants :

3-isobutyl-4-[4-(3-méthyl-but-2-ényl-oxy)-phényl]-furane-2,5-dione,
3-isobutyl-4-[4-(3-méthyl-but-2-ényl-oxy)-phényl]-1H-pyrrole-2,5-dione,
1-hydroxy-3-isobutyl-4-[4-(3-méthyl-but-2-ényl-oxy)-phényl]-1H-pyrrole-2,5-dione,
*3R**,*4S**-1-hydroxy-3-isobutyl-4-[4-(3-méthyl-but-2-ényl-oxy)-phényl]-pyrrolidine-2,5-dione,
*3R**,*4R**-1-hydroxy-3-isobutyl-4-[4-(3-méthyl-but-2-ényl-oxy)-phényl]-pyrrolidine-2,5-dione.

**8.** Mélange tiré d'un mycélium de *Antrodia camphorata,* pour

a) faire baisser la pression sanguine,
b) faire augmenter le taux de lipoprotéines de haute densité,
c) induire un agonisme cholinergique central;
d) fournir au foie une protection contre la D-galactosamine ou le virus de l'hépatite C,
e) ou traiter une inflammation,

pour lequel le mycélium a été préalablement préparé par fermentation submergée en milieu liquide, et lequel mélange a été préparé à partir d'un extrait dudit mycélium dans l'eau ou dans un solvant organique.

**9.** Mélange conforme à la revendication 8, pour lequel le solvant organique est un alcool, un ester, un alcane ou un alcane halogéné.

**10.** Mélange conforme à la revendication 9, pour lequel l'alcool est de l'éthanol.

**11.** Mycélium de *Antrodia camphorata,* pour

a) faire baisser la pression sanguine,
b) faire augmenter le taux de lipoprotéines de haute densité,
c) induire un agonisme cholinergique central;
d) fournir au foie une protection contre la D-galactosamine ou le virus de l'hépatite C,
e) ou traiter une inflammation,

lequel mycélium a été préparé par fermentation submergée en milieu liquide.

**12.** Composé conforme à la revendication 1, ou composition conforme à la revendication 6, l'agonisme cholinergique central devant servir à traiter ou prévenir la maladie d'Alzheimer.

**13.** Utilisation d'un composé conforme à la revendication 1, d'une composition conforme à la revendication 6, d'un mélange conforme à la revendication 8, ou d'un mycélium conforme à la revendication 11, en vue de la fabrication d'un médicament conçu pour

a) faire baisser la pression sanguine,
b) faire augmenter le taux de lipoprotéines de haute densité,
c) induire un agonisme cholinergique central;
d) fournir au foie une protection contre la D-galactosamine ou le virus de l'hépatite C,
e) ou traiter une inflammation.

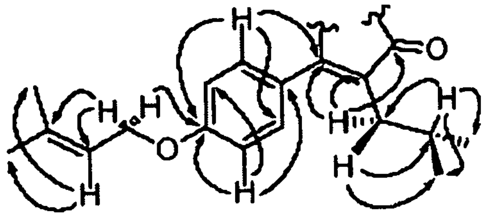

Figure 1

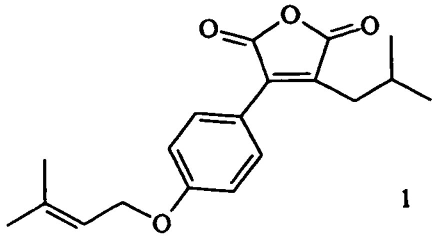

1

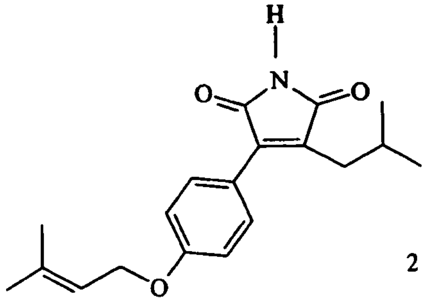

2

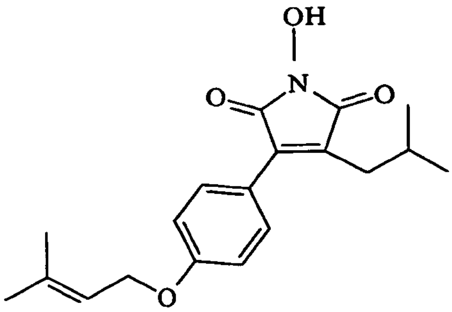

3

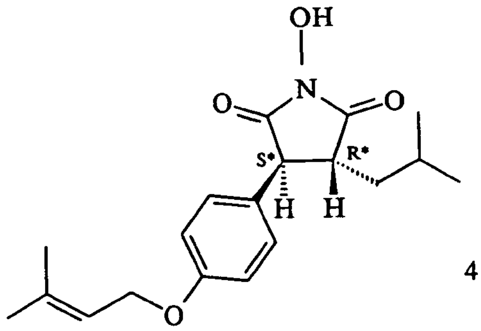

4

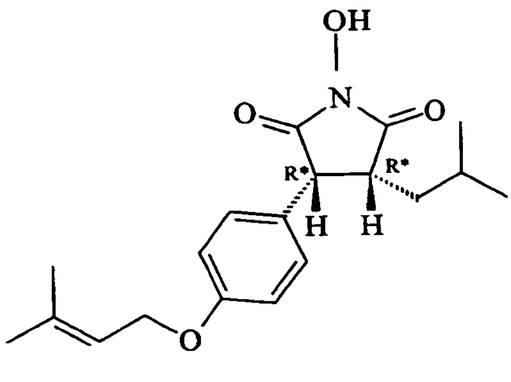

5

Figure 2

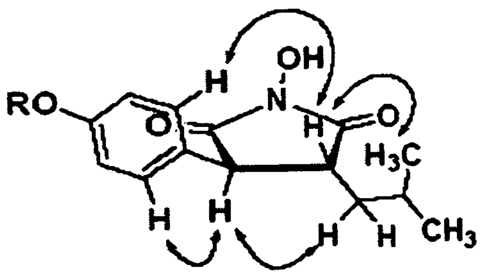

4

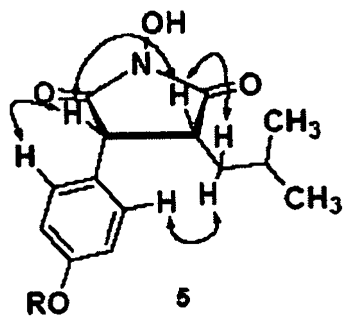

5

Figure 3

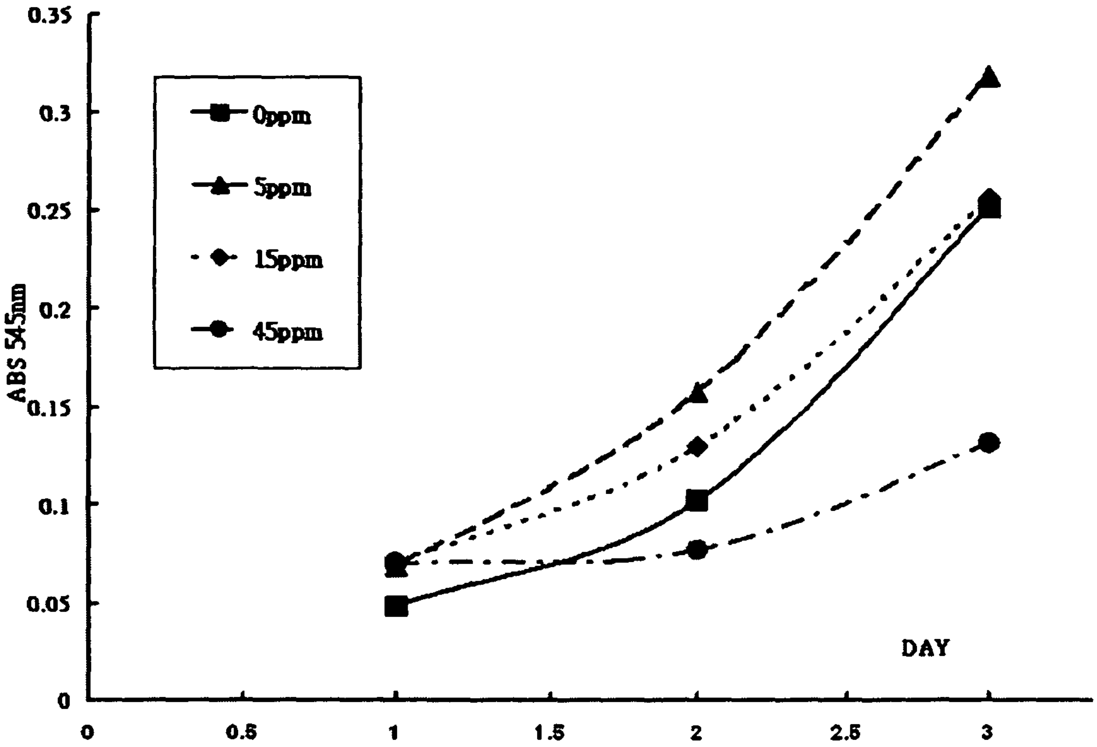


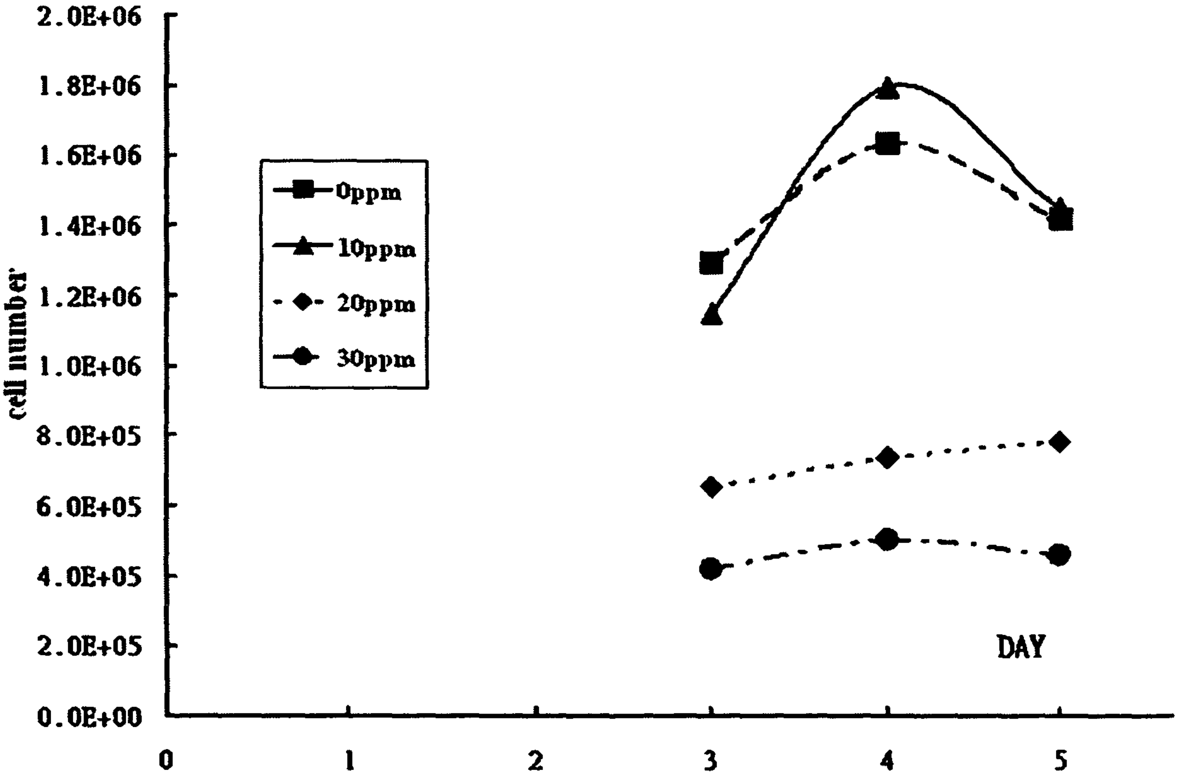


Figure 4-a (upper) and Figure 4-b (down)

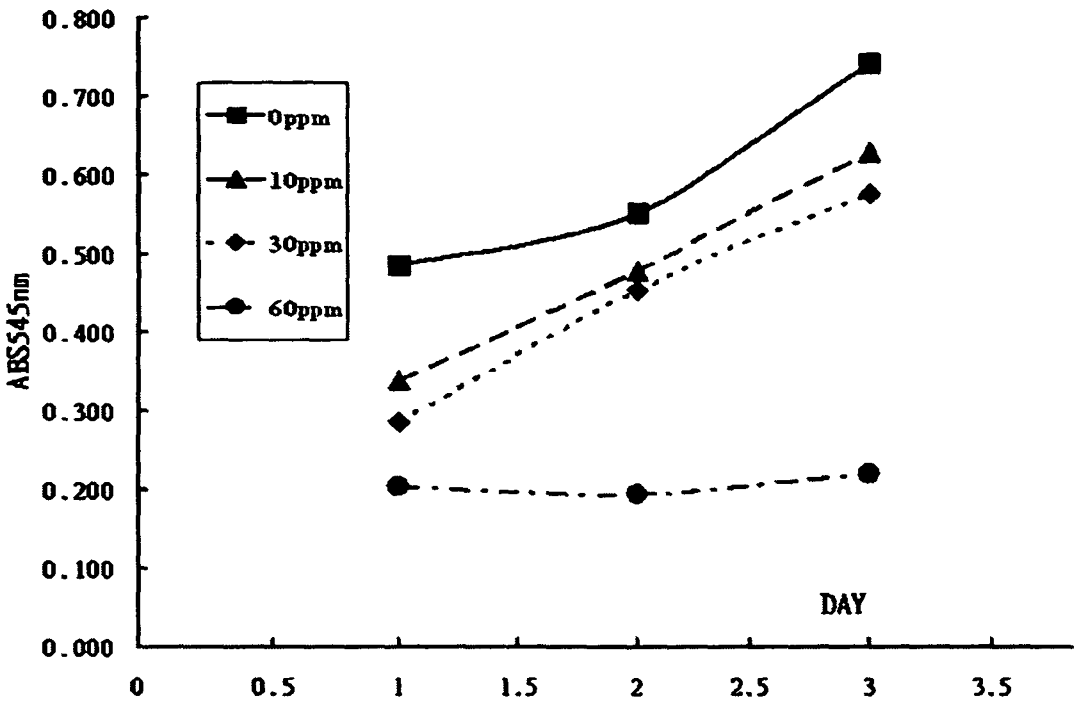


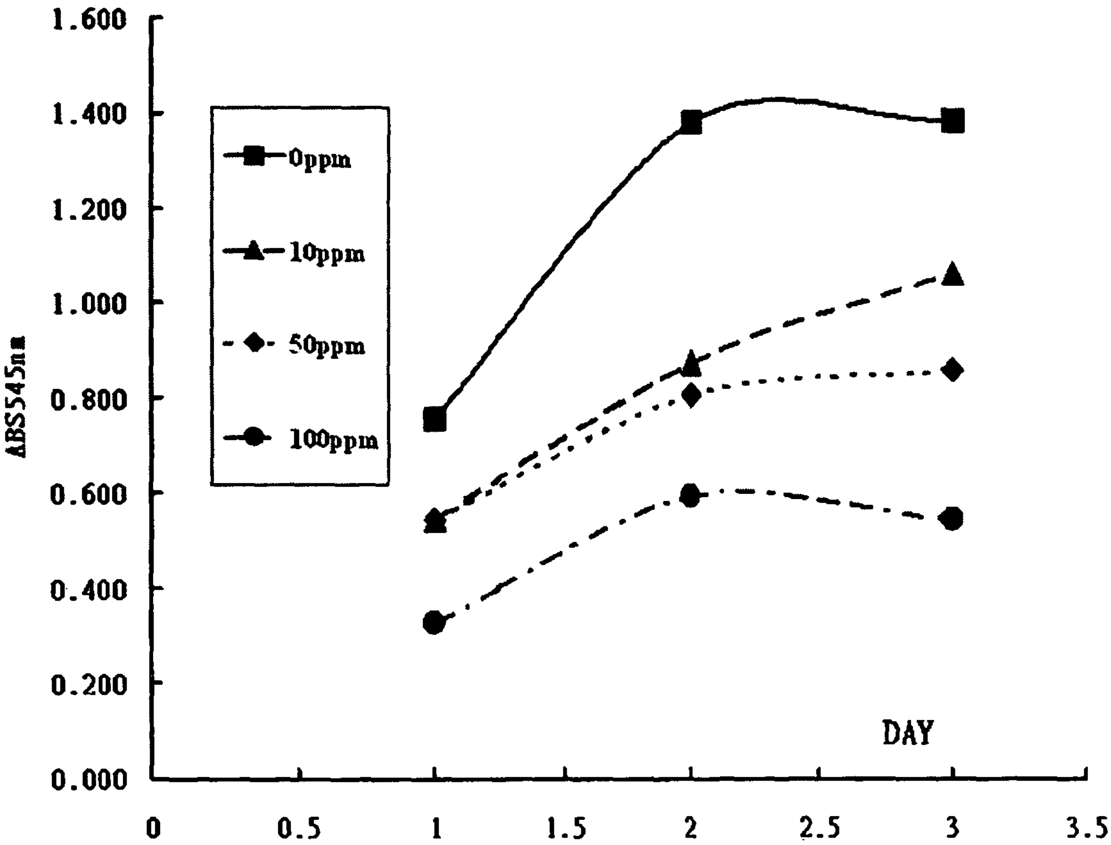


Figure 4-c (upper) and Figure 4-d (down)

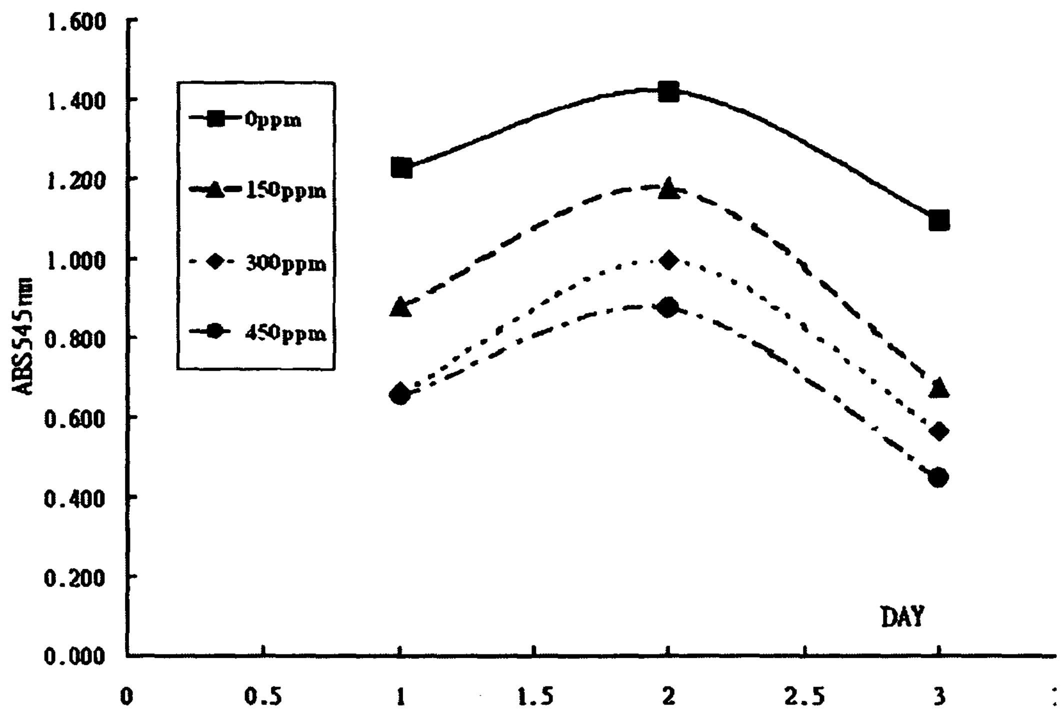


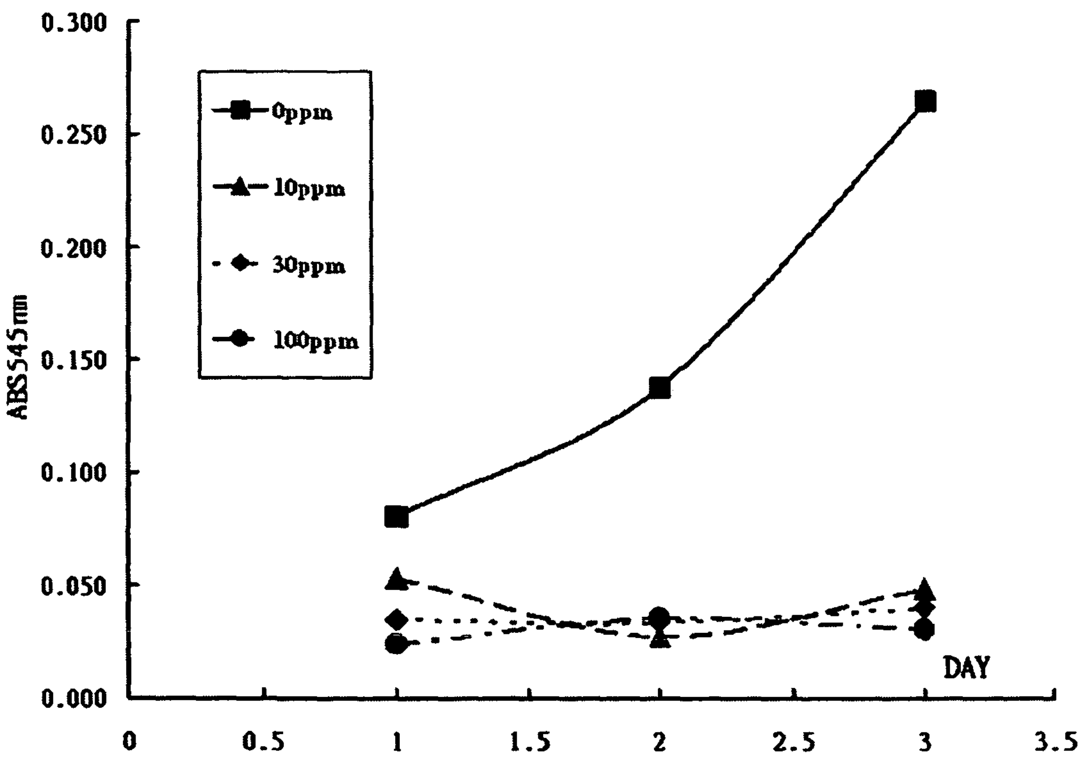


Figure 5-a (upper) and Figure 5-b (down)

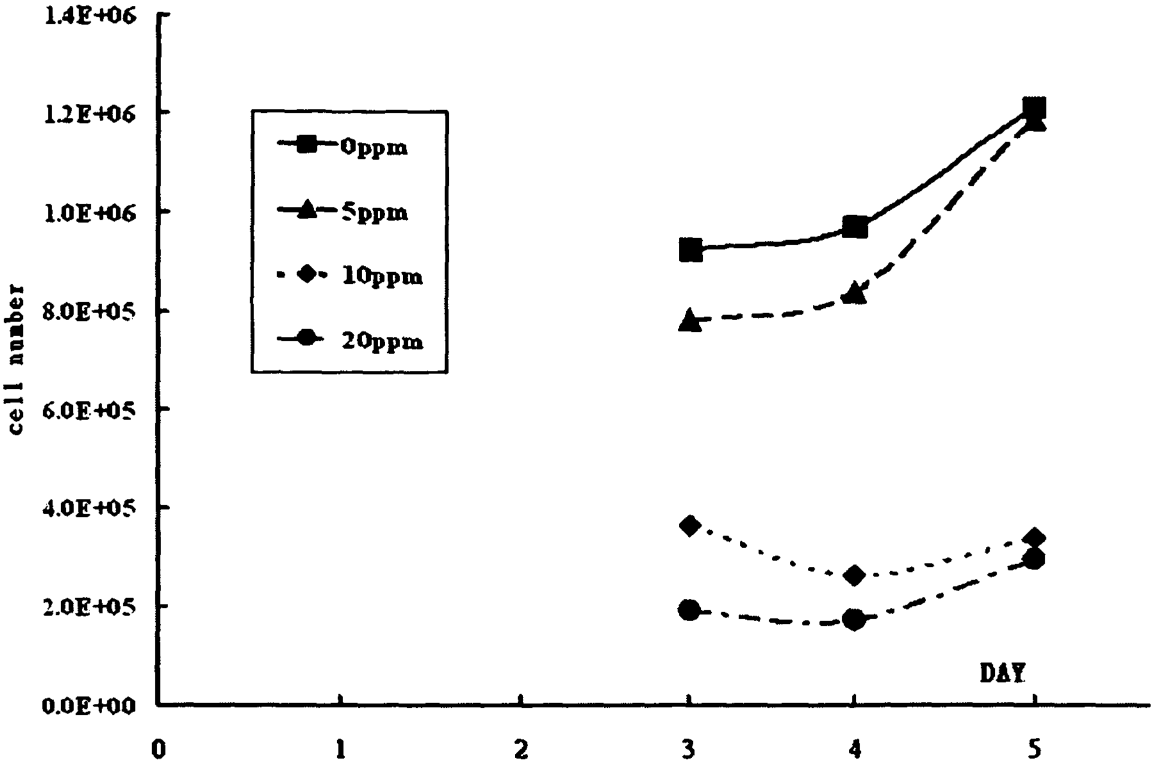
1.4E+06
1.2E+06
1.0E+06
8.0E+05
6.0E+05
4.0E+05
2.0E+05
0.0E+00
cell number
0ppm
5ppm
10ppm
20ppm
DAY
0
1
2
3
4
5

Figure 5-c

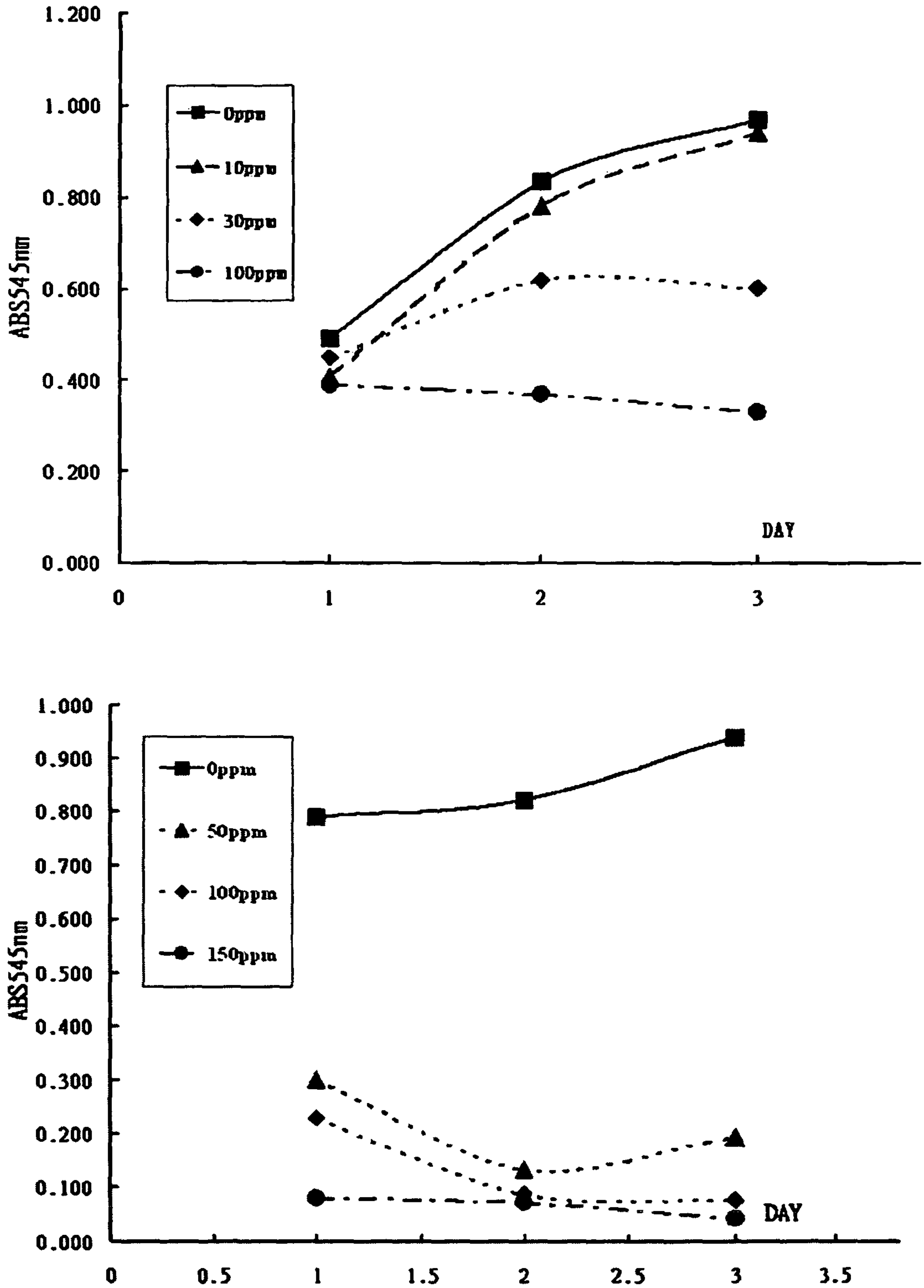


Figure 6-a (upper) and Figure 6-b (down)

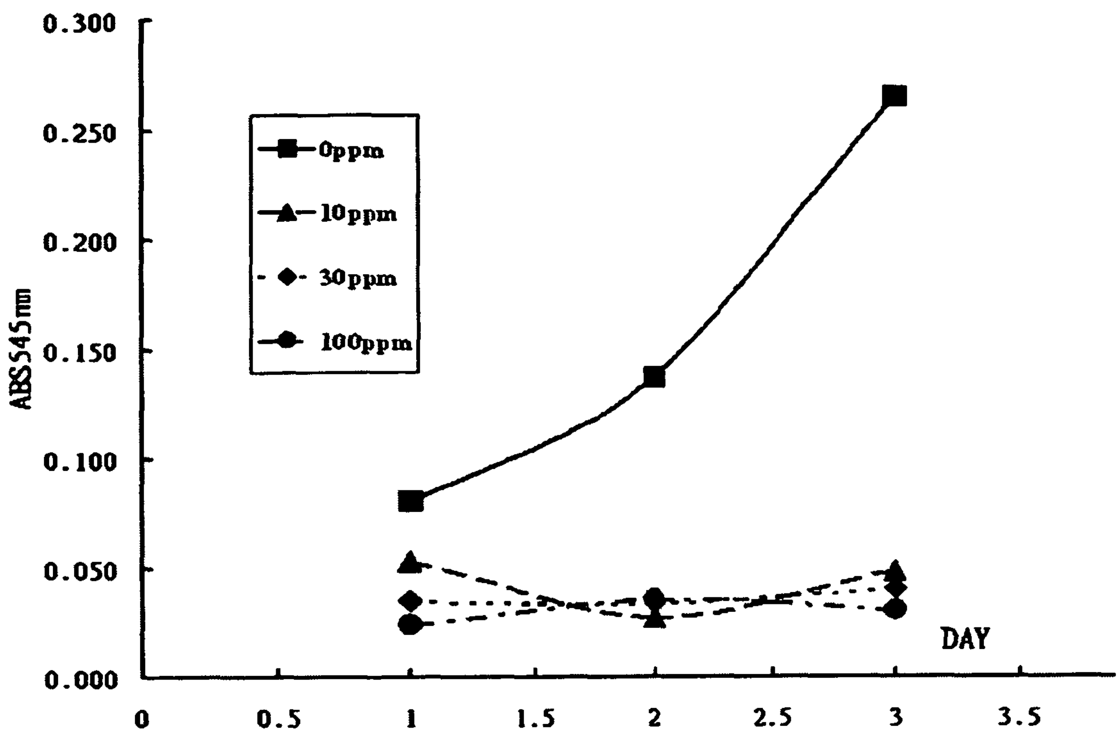


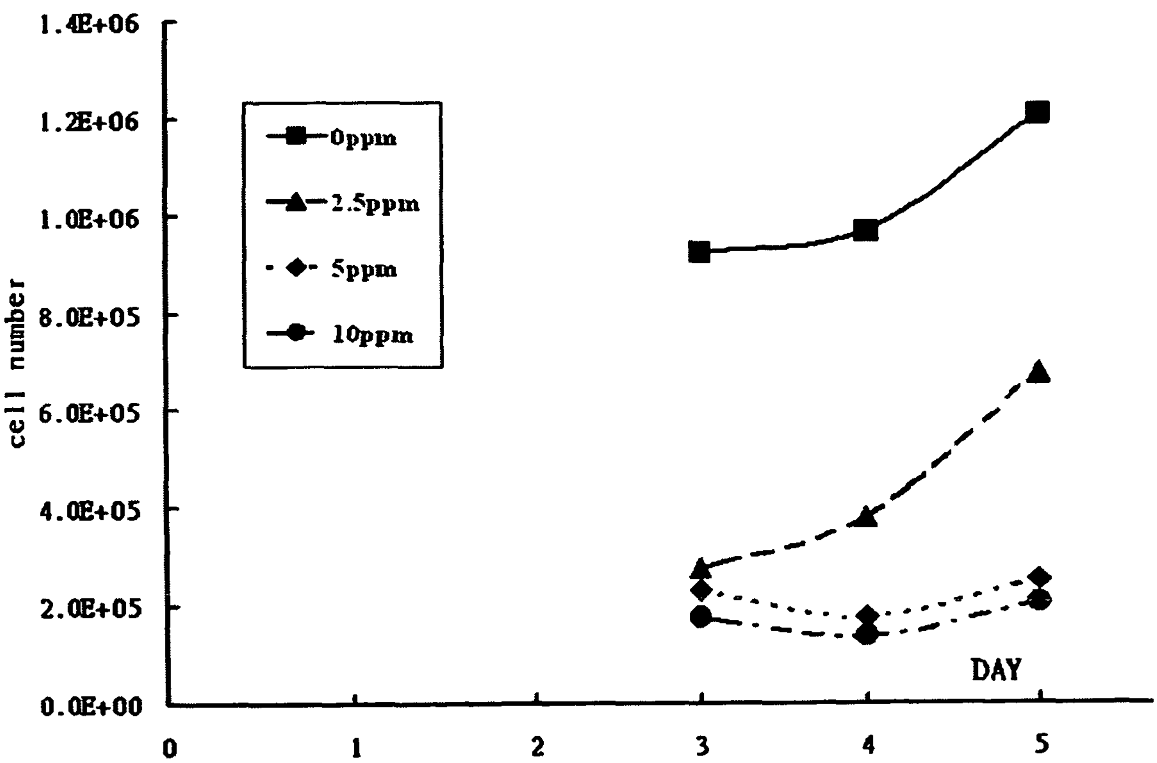


Figure 6-c (upper) and Figure 6-d (down)

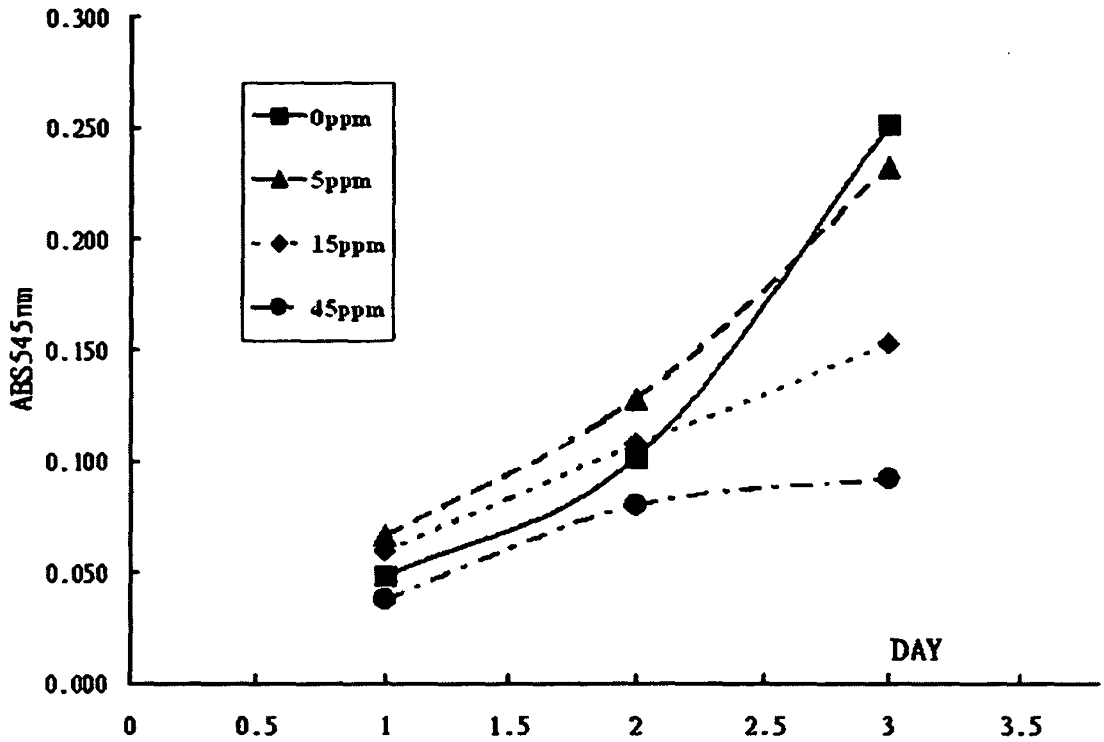


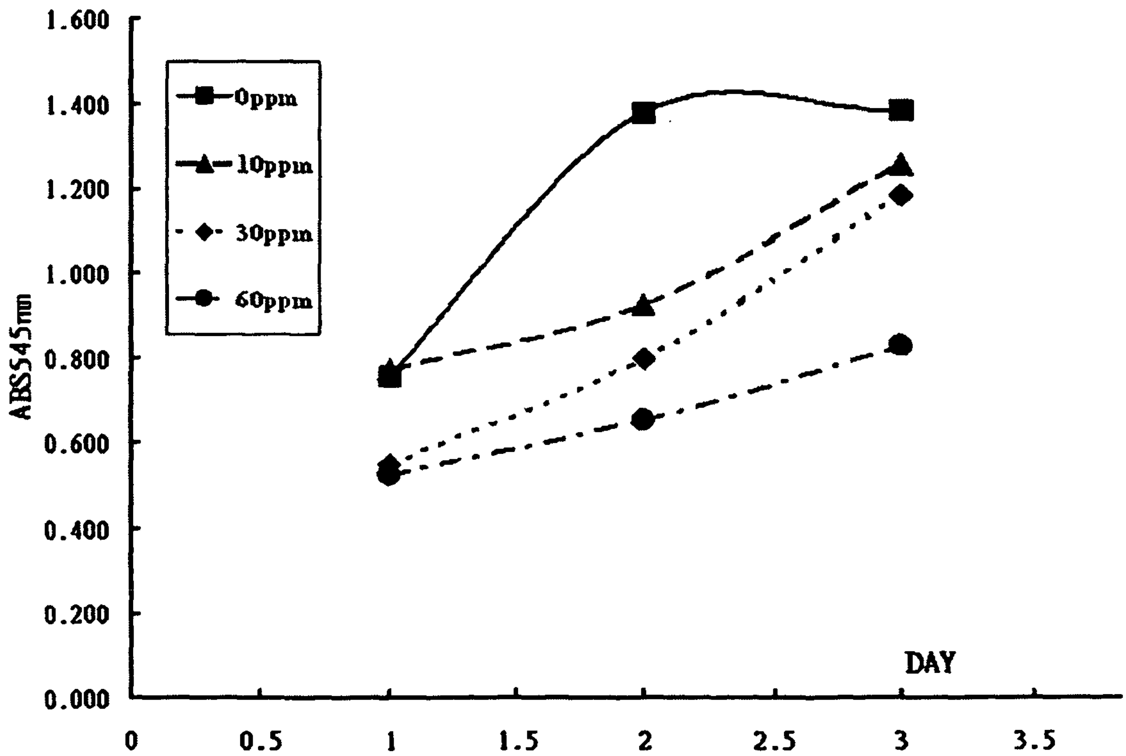


Figure 6-e (upper) and Figure 6-f (down)

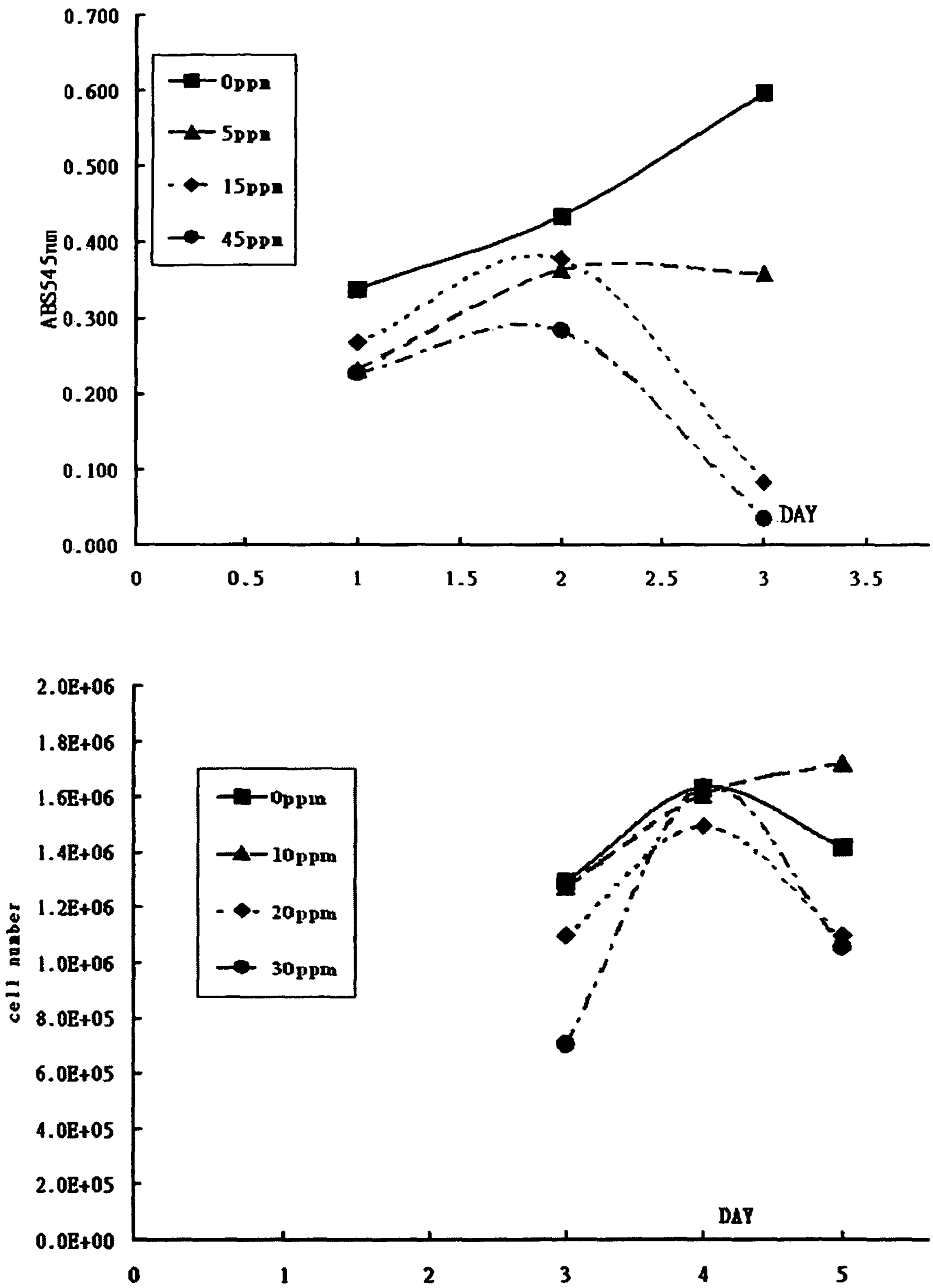


Figure 7-a (upper) and Figure 7-b (down)

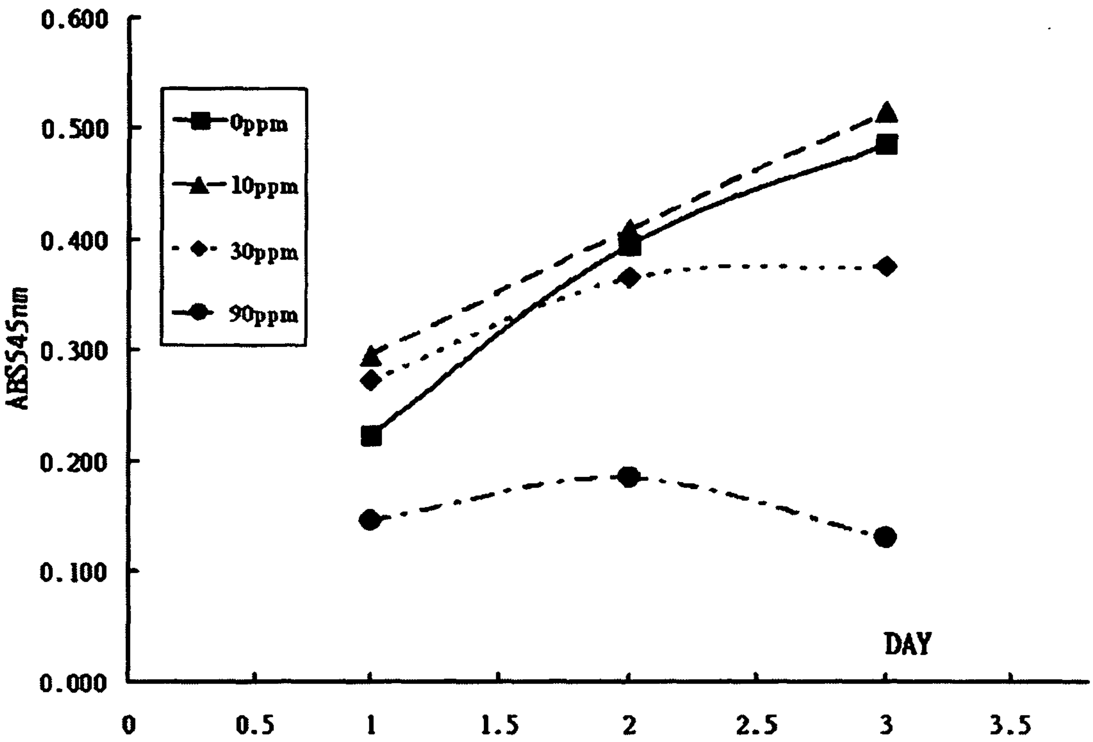


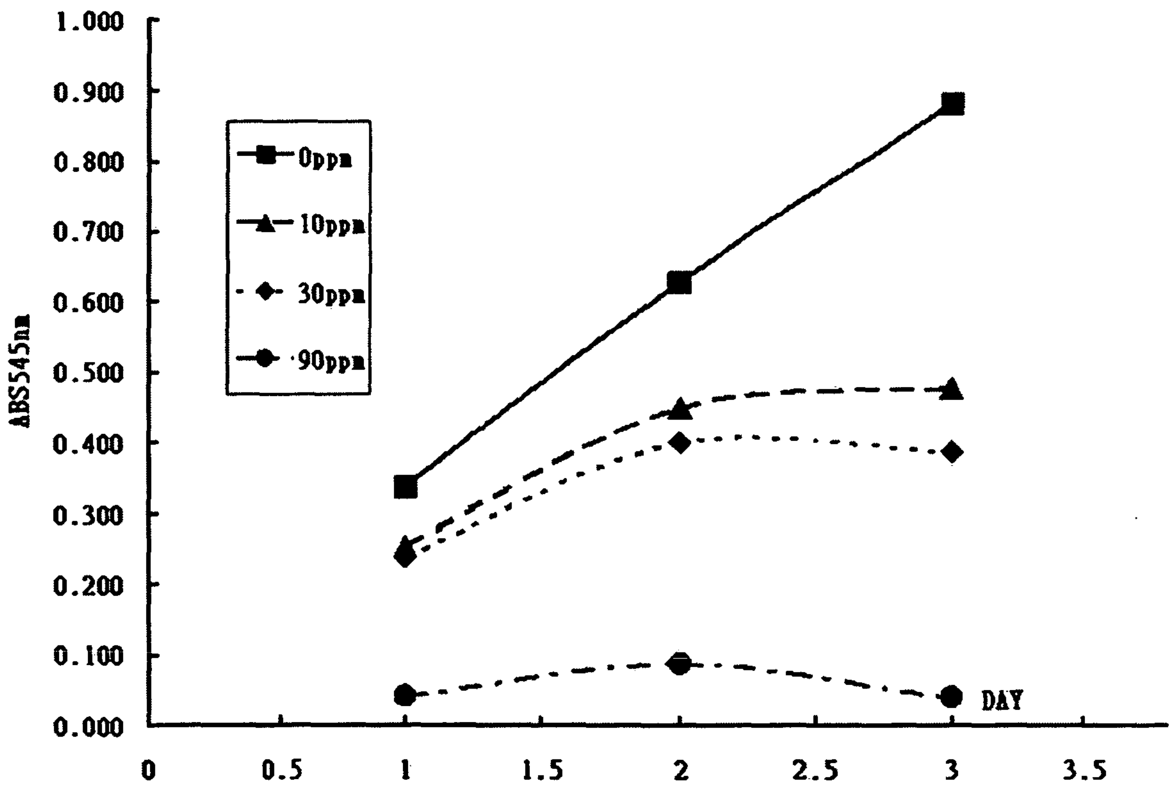


Figure7-c (upper) and Figure7-d (down)

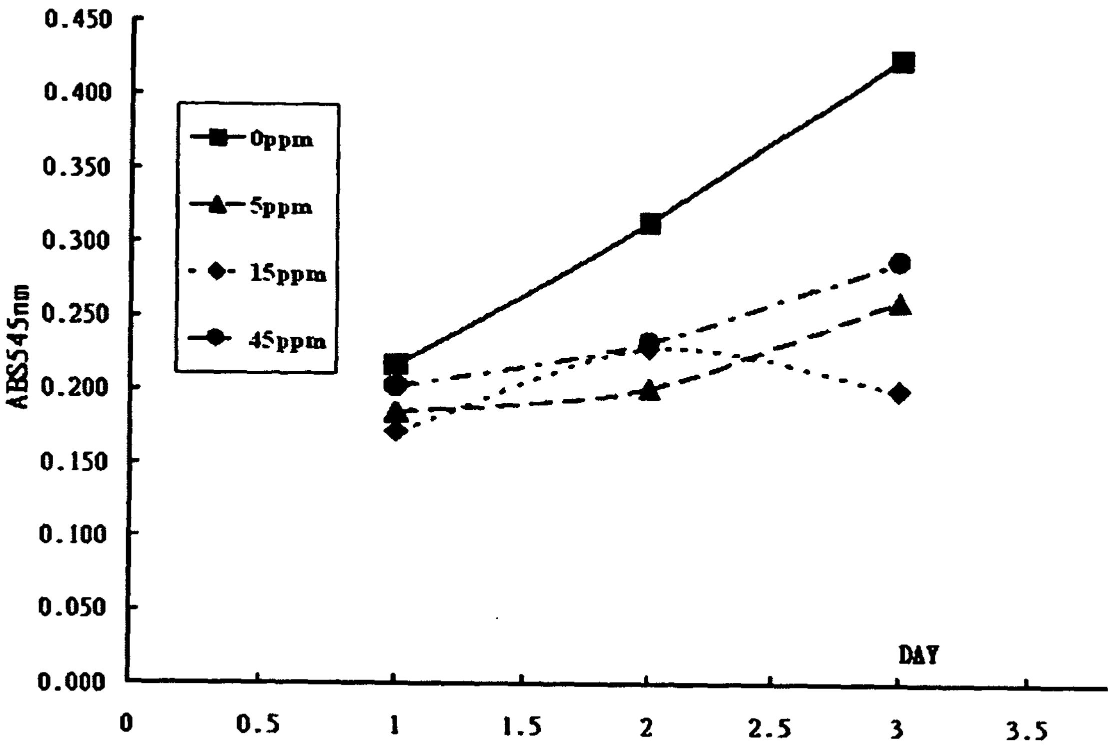
0.450
0.400
0.350
0.300
0.250
0.200
0.150
0.100
0.050
0.000
ABS545nm
0ppm
5ppm
15ppm
45ppm
DAY
0
0.5
1
1.5
2
2.5
3
3.5

Figure 7-e

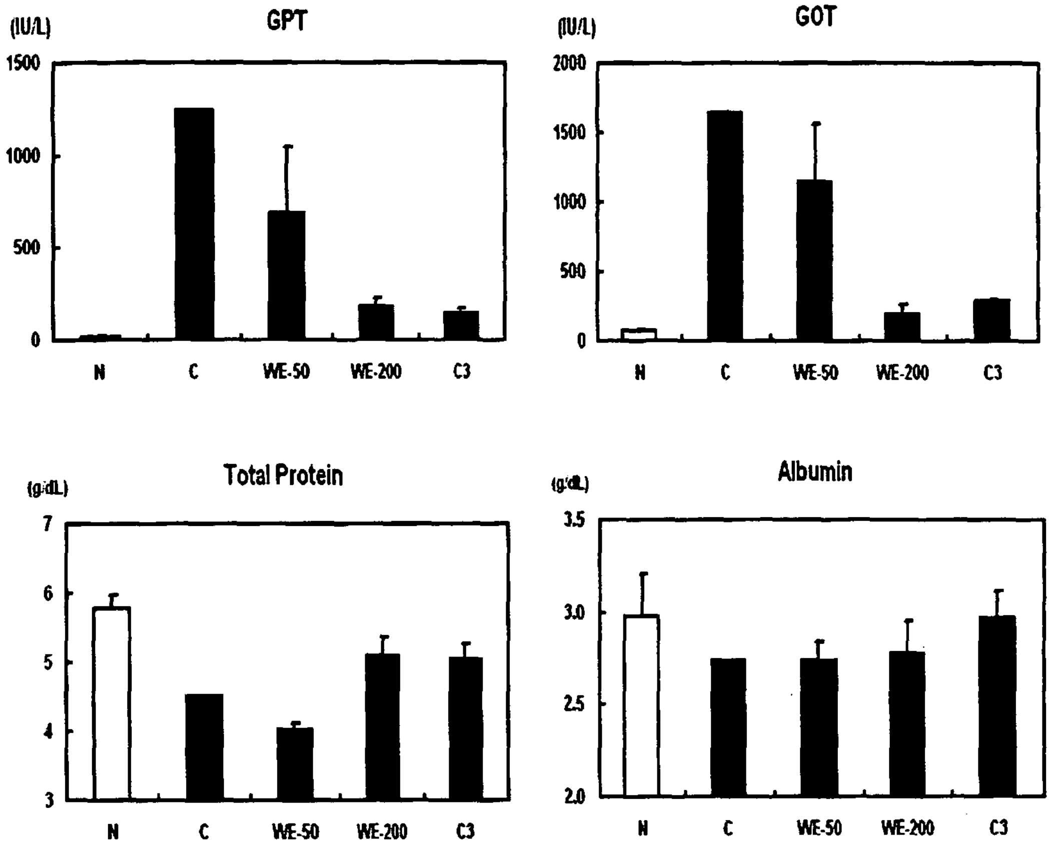
GPT
(IU/L)
1500
1000
500
0
N
C
WE-50
WE-200
C3
GOT
(IU/L)
2000
1500
1000
500
0
N
C
WE-50
WE-200
C3
Total Protein
(g/dL)
7
6
5
4
3
N
C
WE-50
WE-200
C3
Albumin
(g/dL)
3.5
3.0
2.5
2.0
N
C
WE-50
WE-200
C3

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2004092000 A
- US 2003113297 A
- US 2003148517 A
- GB 1560464 A
- US 4188398 A

### Non-patent literature cited in the description


- **Wu, S.-H. et al.** Antrodia cinnamomea (''niu-chang-chih''), New combination of a medicinal fungus in Taiwan. *Bot. Bull. Acad. Sin.,* 1997, vol. 38, 273-275
- **NAKAMURA, NORIO et al.** *JOURNAL OF NATURAL PRODUCTS,* 2004, vol. 67 (1), 46-48
- **TOMINAGA, YOSHINORI et al.** *JOURNAL OF HETEROCYCLIC CHEMISTRY,* vol. 39 (3), 571-59
- **WIJNBERG, J. B. P. A. et al.** *TETRAHEDRON,* 1978, vol. 34 (2), 179-87
- **MAU et al.** *FOOD CHEMISTRY,* June 2004, vol. 86, 25-31
- **DAI et al.** *JOURNAL OF FOOD AND DRUG ANALYSIS,* 2003, vol. 11 (3), 177-185
- **HSEU et al.** *LIFE SCIENCES,* 2002, vol. 71, 469-482
- **Berge et al.** Pharmaceutically Acceptable Salts. *J. Pharm. Sci.,* 1977, vol. 66, 1-19
- **Lisa R. Fodero ; David H. Small.** Cholinergic abnormalities in Alzheimer's disease: are there new targets for drug development?. *Drug Development Research,* 2002, vol. 56 (3), 369-379
- **T. L. M. Stamford et al.** Protein enrichment of cashew wastes for animal feeds. *Food Science, http://www.unu.edu/unupress/food/8F101e/8F101E0b.htm.*